# EUROPEAN PATENT APPLICATION

(11) **EP 3 973 925 A1**
(43) Date of publication of application: **30.03.2022**
(21) Application number: 21020060.6
(22) Date of filing: 05.02.2021
(51) Int. Cl.: A61F 2/24, A61B 17/12, A61F 2/966, A61F 2/95

(54) **APPARATUS FOR TREATING CARDIOVASCULAR VALVE DYSFUNCTION**

(30) Priority: 23.09.2020 EP 20020429
(71) Applicant: Verine, Vitali, 1207 Geneva (CH); Romoscanu, Alexandre, 1207 Geneva (CH); Delaloye, Stéphane, 8180 Bülach (CH)
(72) Inventor: Verine, Vitali, 1207 Geneva (CH); Romoscanu, Alexandre, 1207 Geneva (CH); Delaloye, Stéphane, 8180 Bülach (CH)

(57) **Abstract**

A technique for treating or relieving a dysfunctional mitral valve by implanting prosthetic valves (10) in pulmonary veins (PV), optionally at or close to the ostium (VO). The prosthetic valves may function upstream of the mitral valve, to reinforce the mitral valve function and/or to mitigate effects of mitral valve dysfunction. Specific valves suitable for pulmonary vein implantation are described. Also described is a retrieval technique for retrieving and recollapsing a cardiovascular implant such as a cardiovascular valve after implantation. Also described is a technique of release and repositioning of a cardiovascular valve using the retrieval means.

## Description

### Field of the Disclosure

The present disclosure relates to prosthetic devices for treating or mitigating the effects of dysfunctional cardiovascular valves. Certain non-limiting aspects focus on dysfunctional cardiac valves, especially but not limited to a dysfunctional mitral valve.

### Background to the Disclosure

Dysfunctional cardiovascular valves, especially cardiac valves, reduce the efficiency with which blood circulates in the body. Blocked or narrowed valves reduce forward flow. Leaky valves allow reverse flow. Dysfunctional valves may be caused by heart diseases or by other causes, but the net result is that the heart has to work increasingly harder to circulate blood to support bodily function.

Traditional approaches for treating dysfunctional cardiac valves require the cutting of a relatively large opening in the patient's sternum ("sternotomy") or thoracic cavity ("thoracotomy") in order to allow a surgeon to access the patient's heart. Additionally these approaches require the arrest of the patient's heart and a cardiopulmonary bypass. In recent years, efforts have been made to reduce invasiveness by using a transcatheter procedure, namely by delivering and implanting a prosthetic device, for example a replacement valve, via a catheter inserted through a smaller skin incision, using either a transvascular route or a transapical route to the implantation site. The prosthetic device is delivered in a collapsed condition by the catheter, and is expanded or self-expands at the implantation site into its implanted state. Such a prosthetic valve is referred to as a stent-valve or a valved stent.

Stent-valves designed for implantation at the aortic valve position have been successfully developed and are routinely implanted in suitable patients, for example, elderly or frail patients suffering from aortic stenosis. However, stent-valves for the mitral valve position have proven more challenging to develop successfully. The native mitral valve anatomy is very different from and more complex than the aortic valve. The native aortic valve basically comprises three passive leaflets at the left ventricle outflow tract that have to withstand modest back-pressure of blood during diastole. Also calcification typically present at the native aortic valve aids anchoring of the prosthetic valve by a friction fit within the native aortic valve. In contrast, the native mitral valve is larger and more complex; extends into and is partly supported from within the ventricle; and has to withstand higher blood back-pressure during systole. Delivering a large prosthetic valve via a small catheter is problematic. It is difficult to compress a large valve to a very small size suitable for catheterization without risking damage to the prosthetic valve. Even a small amount of damage can impact valve durability. Alternatively, using a large diameter catheter limits use only to patients having a suitable vasculature, and makes the procedure more invasive. A further consideration is that the complex nature of the mitral valve apparatus makes the prosthesis more vulnerable to biological effects, such as thrombus formation. Moreover, it is not possible to anchor a prosthetic valve with a simple friction fit within the native mitral valve, because the risk of the prosthesis dislodging or migrating would be too high. Prosthetic mitral valves require additional anchoring, risking interference with other anatomical structures of the heart. The above considerations also make it very difficult to envisage such a prosthetic valve that can be re-captured or re-compressed in situ in the body after implantation, to enable the valve to be removed by a catheter when desired (for example, for replacement in a later procedure, or for temporary implantation).

It would be desirable to address and/or mitigate one or more of the aforementioned issues.

### Summary of the Disclosure

The following presents a simplified summary of the disclosure in order to provide a basic understanding of some aspects of the disclosure. This summary is not an extensive overview of the disclosure. It is intended to neither identify key or critical elements of the disclosure nor delineate the scope of the disclosure. Its sole purpose is to present some concepts of the disclosure in a simplified form as a prelude to the more detailed description that is presented later.

One aspect of the disclosure relates to a technique for treating or relieving a dysfunctional mitral valve. The disclosure provides at least one prosthetic valve configured for implantation in a pulmonary vein (optionally at or close to the ostium, for example at a segment of the vein in proximity to the ostium). The prosthetic valve may function upstream of the mitral valve, to reinforce the mitral valve function and/or to mitigate effects of mitral valve dysfunction. For example, the prosthetic valve may close to prevent undesired back-flow of blood out of the heart during ventricular systole, and to reduce exposure of the pulmonary vein to the high ventricular systolic blood pressure. In turn, this can prevent pulmonary hypertension problems relating to pulmonary venous hypertension.

Implantation of the prosthetic valve in a pulmonary vein may offer different perspectives from implantation at the mitral valve position. The pulmonary vein is smaller in diameter and/or cross-section than the mitral annulus. A smaller prosthetic device may be used than at the mitral position. A smaller size can facilitate compression into a small-diameter delivery catheter, and facilitate access to the implantation site via narrow vasculature. The forces exerted on a smaller valve by blood under systolic pressure are lower, enabling the valve to the held in place by lower anchoring forces than at the mitral position. It also becomes practical to envisage a prosthetic valve that can be re-collapsed and retrieved after implantation.

Although a single prosthetic valve may be provided for implantation, it is envisaged to provide plural (e.g. at least two, at least three, at least four, or five) prosthetic valves for implantation in respective different pulmonary veins (e.g. at or close to the ostia). The function of the mitral valve is thereby distributed across sites of plural pulmonary veins. A prosthetic valve for each of the respective pulmonary veins may provide most complete valve function to prevent back-flow of blood out of the heart, and protect all of the pulmonary veins from exposure to high systolic blood pressure. Most patients have four pulmonary veins, but it is not uncommon for a patient to have five pulmonary veins.

Implantation of prosthetic valves in one or more pulmonary veins also has advantages in avoiding interfering with the native mitral valve and surrounding accesses, allowing future surgical treatment of the mitral valve apparatus if needed, as well as future minimally invasive and percutaneous techniques. This could be very useful in certain clinical situations when the mitral valve repair or replacement is impossible or undesirable, for example, due to multiple morbidities. Example situations may be acute severe mitral regurgitation complicating acute myocardial infarction or mitral valve endocarditis causing hemodynamic compromise. In such clinical scenarios, the implantation of the stent-valves into pulmonary veins could temporarily stabilize patient's hemodynamic compromise. Surgery or percutaneous intervention to correct the mitral valve function could be performed at a later time-point (for example, 3 to 4 weeks after implantation during the acute phase). In this regard the possibility optionally to retrieve previously implanted pulmonary vein valves percutaneously or during the surgical procedure becomes highly advantageous.

Valves designed for implantation at traditional cardiac valve sites may not automatically be suitable for use in a pulmonary vein. The pulmonary vein does not support any native valve, and so does not have any naturally reinforced annulus tissue, contrary to native cardiac valve sites which do have naturally reinforced annulus tissue. Instead, the pulmonary vein wall is somewhat elastic. Also, the vein wall is thinner than, for example, an aortic wall and so less adapted to withstand substantial anchoring forces or be engaged by aggressive anchoring profiles. A yet further consideration is that the pulmonary vein comprises multiple tributary vessels leading from the lung into the main vein vessel, even close to the ostium. Avoiding occlusion of these tributary vessels is important for maintaining good perfusion of blood through the lung tissue, and free flow of oxygenated blood from the lungs to the heart for circulation to support bodily function.

Careful design of a prosthetic valve adapted to the unique characteristics of the pulmonary vein is a significant part of the present disclosure. Suitable valve designs are described in various aspects below. These aspects may be independent, but are explicitly envisaged in combination with the present aspect, and advantageously in combination with each other and the present aspect.

A closely related aspect of the present disclosure provides a prosthetic cardiovascular valve (prosthesis), configured to be delivered to an implantation site in a collapsed condition by a delivery catheter, the prosthesis being configurable reversibly between a collapsed condition and an expanded condition, the prosthesis comprising a plurality of attachment elements for removable engagement with a holder of the delivery catheter for facilitating positioning and deployment of the prosthesis, and at least one flexible retrieval element for facilitating retrieval of the prosthesis after deployment.

In use, the plurality of attachment elements may serve to removably engage a holder of the delivery catheter used to deliver the prosthesis in its collapsed condition. The attachment elements may have the form of one or more of: projections (e.g. T-shaped, or L-shaped, mushroom-head shaped, or diamond-shaped), hooks, crotchets, paddles, apertures (e.g. round, oval, or C-shaped). The attachment elements may prevent axial displacement of the prosthesis with respect to the holder of the delivery catheter, at least in one axial direction, while the attachment elements remain engaged with the holder. In the case of a self-expanding prosthesis, the attachment elements may prevent the prosthesis from tending to pop-out prematurely from the delivery catheter during the deployment procedure.

After deployment, and optionally after release of the attachment elements from the holder of the delivery device, the flexible retrieval element may serve to facilitate retrieval of the deployed prosthesis, either by means of the same delivery catheter or by means of a different retrieval catheter. The flexible retrieval element may present an exposed elongate section, for example suspended between the attachment elements, suitable for being hooked for retrieval.

The flexible retrieval element may be configured such that applying tension to the flexible retrieval element, for example in an axial direction, tends to contract at least a portion of the prosthesis adjacent to the flexible retrieval element, to facilitate drawing the prosthesis into a retrieval catheter. For example, the flexible retrieval element may be suspended at plural positions around the circumference of the prosthesis. Pulling the flexible retrieval element in an axial direction (optionally while countering axial force on the prosthesis in order to prevent unwanted axial movement of the prosthesis in the pulmonary vein before adequate prosthesis contraction) tensions the element, and tends to draw the plural positions circumferentially and/or radially closer to one another.

The flexible retrieval element may be positioned at or towards an outflow end of the prosthesis. In the case of a pulmonary vein prosthesis, the outflow end corresponds to the end that is closest to and/or may extend partly within, the ostium and/or the left atrium.

The flexibility of the retrieval element may avoid the retrieval element from interfering with the collapsibility of the prosthesis.

In some examples, the region of the stent supporting the flexible retrieval element (e.g. the attachment elements) may be positioned inwardly or canted inwardly with respect to a trunk portion of the stent when the stent is in the expanded condition. This may space the flexible retrieval element radially inwardly of the surrounding vessel wall, to facilitate hooking by a retrieval catheter. An inwardly canted shape may also facilitate retrieval and collapsing to the collapsed condition by presenting an initial taper to facilitate sliding a collapsing sheath over the prosthesis starting at a canted-in end.

The flexible retrieval element may, for example, comprise or be any one or more of: a filament, thread (single strand or multi-strand), fibre, wire, chain, and/or coil. Example materials include suitable polymers, and/or metals (e.g. steel or nitinol).

The plurality of attachment elements may include at least a first sub-set (e.g. of at least one attachment element) and a second sub-set (e.g. of at least one attachment element) arranged at (or having attachment features arranged at) respective different first and second positions along an axis of the stent, at least in the collapsed condition of the stent (and optionally also in the expanded condition). For example, the first and second sub-sets may have projections (e.g. T-shaped, or L-shaped, mushroom-head shaped, or diamond-shaped), hooks, crotchets, paddles, apertures (e.g. round, oval, or C-shaped) at different axial positions and/or axially offset in one sub-set with respect to another sub-set. The first and second sub-sets of attachment elements are optionally adjacent to, but axially offset from, one another in the axial direction. Such offsetting of the attachment elements and/or attachment features may facilitate collapsing the stent to a smaller diameter, than were all of the attachment elements and/or attachment features to be arranged at the same axial position. Offsetting can avoid the attachment elements from interfering with one another when the stent is converted to or held in its collapsed condition. Looking from a different perspective, it can also enable a larger number of attachment elements and/or attachment features to be used without compromising the diameter of the stent when in its collapsed condition.

If desired, the first and second sub-sets may have a shape or configuration that interfit with respect to one another, and /or nestle together, when in the collapsed condition. The disposition of attachment elements may alternate in the circumferential direction between the first and second sub-sets, such that each attachment element of the first sub-set is disposed circumferentially between two attachment elements of the second sub-set.

Additionally or alternatively to the above, a closely related aspect of the present disclosure provides a prosthetic cardiovascular valve (prosthesis), configured to be delivered to an implantation site in a collapsed condition by a delivery catheter, the prosthesis being configurable reversibly between a collapsed condition and an expanded condition, the prosthesis comprising a plurality of attachment elements for removable engagement with a holder of the delivery catheter for facilitating positioning and deployment of the prosthesis, and at least one retrieval element for facilitating retrieval of the prosthesis after deployment, the retrieval element having a stowed configuration in which the retrieval element is substantially flush with or flat against a portion of the stent supporting the retrieval element to form a streamlined profile, and a deployed condition in which the retrieval element projects outwardly from the portion of the stent supporting the retrieval element to facilitate engagement by a retrieval device.

The retrieval element may be cantilever mounted to the portion of the stent supporting the retrieval element.

The portion of the stent supporting the retrieval element may be the attachment element.

The retrieval element may be integrally formed with the stent.

The retrieval element may be biased to the deployed configuration, and be deformable to the stowed configuration when the stent is compressed to the collapsed configuration for delivery, the retrieval element self-expanding to the deployed configuration when the stent expands to the expanded configuration.

Additionally or alternatively to any of the above, a closely related aspect of the present disclosure provides a prosthesis (e.g. a prosthetic cardiovascular valve), configured to be delivered to an implantation site in a collapsed condition by a delivery catheter, the prosthesis being configurable reversibly between a collapsed condition and an expanded condition, the prosthesis comprising at least one flexible filament for use for expansion and/or contraction of the prosthesis, the filament being an integral part of the prosthesis, for example, such that the filament remains integrally captive to the prosthesis after implantation.

As well as, or as an alternative to, providing a retrieval feature as described earlier, the flexible filament can also be used for compressing the stent-valve to allow its easier placement into delivery catheter. The flexible retrieval element could also serve as the last attachment of the prosthesis to delivery catheter before the prosthesis is completely released providing possibility to re-collapse the prosthesis for resheathing and repositioning to a better location if an initial position in the vessel is not optimal. Another advantage of the flexible filament is that during the release of the proximal part of the prosthesis it could act gradually and slowly to prevent jumping of the prosthesis.

By remaining an integral part of the prosthesis, even after implantation, it is not necessary to pull on the flexible filament to remove it after deployment, nor is it necessary to provide removal channels in the prosthesis for allowing the filament to be pulled out. This can provide a more stable implantation procedure than were the filament intended to be withdrawn by being pulled on after deployment, and it can also simplify the design of the prosthesis. Moreover, the presence of at least one filament permanently on the prosthesis can act as a retrieval feature facilitating later retrieval of the prosthesis after implantation.

The prosthesis may include a single such filament, or it may include plural filaments at, or acting at, different axial positions along the length of the prosthesis.

Additionally or alternatively to any of the above, a closely related aspect of the present disclosure provides a prosthesis (e.g. a prosthetic cardiovascular valve), configured to be delivered to an implantation site in a collapsed condition by a delivery catheter, the prosthesis being configurable reversibly between a collapsed condition and an expanded condition, the prosthesis comprising a generally tubular stent including at least a trunk portion and at least one antimigration element carried by the trunk portion, wherein the antimigration element has a shape configured to resist migration of the stent after implantation in body tissue, and the antimigration element does not protrude radially outwardly compared to an outer surface of the trunk portion adjacent the antimigration element.

The antimigration element may have the non-protruding configuration at least in the collapsed condition of the stent, but optionally also in the expanded condition.

In some embodiments, the antimigration element has a radially outermost portion that lies radially inwardly, by at least a certain distance, of a radially outwardly facing surface of the trunk portion adjacent the antimigration member. The radial distance may, for example, be at least 0.1 mm, optionally at least 0.2mm, optionally at least 0.3mm, optionally at least 0.4mm, optionally at least 0.5mm, optionally at least 0.6mm, optionally at least 0.7mm, optionally at least 0.8mm, optionally at least 0.9mm, optionally at least 1mm.

Disposing the antimigration element so that it does not protrude radially outwardly compared to an outer surface of the trunk portion adjacent the antimigration element (and optionally is spaced radially inwardly by at least a certain distance), avoids the antimigration element interfering with operation of a delivery catheter for introducing the prosthesis to an implantation site, and/or a retrieval catheter for retrieving and/or re-sheathing the prosthesis after implantation. Such a catheter typically may comprise a sheath that slides directly over and in contact with the prosthesis outer surface in its collapsed condition. Problem-free operation of the catheter may rely on the sheath being able to slide, without too much friction, over the surface of the prosthesis at the distal end of the catheter. The disposition of the antimigration element so that it does not protrude radially, and hence does not interfere with the sheath is a significant advantage.

In some embodiments, the (or each) antimigration element is coupled independently to the adjacent trunk portion of the stent. The trunk portion may provide a certain radial stiffness for the stent. The antimigration element may move with the trunk portion that supports it. The antimigration element may optionally protrude, in an axial direction, from the adjacent trunk portion of the stent. The antimigration element may optionally protrude, in an axial direction, from an axial extremity of the trunk portion. The antimigration element may optionally be disposed at (and may optionally define) an end extremity of the stent and/or prosthesis.

The antimigration element may, for example, comprise an antimigration feature, for example, a barb and/or prong, for penetrating at least partly into anatomical tissue in contact with the antimigration member when the prosthesis is implanted. The antimigration element may comprise a cantilever support having a first coupled end, coupled to (e.g. extending from) the trunk portion of the stent. The cantilever support may be canted, or otherwise project, at least partly radially inwardly away from the first end. The feature may be carried at a second free end of the cantilever support and be bent, curved or angled radially outwardly compared to the cantilever support. The inward canting of the cantilever support can provide sufficient radial space to accommodate the radial height of the barb or prong, without the barb or prong projecting radially proud of the outer surface of the trunk portion.

The stent may be a self-expanding stent or it may be plastically expandable. The antimigration members are especially suitable when the stent is a self-expanding type that exerts a radially outward force against the catheter sheath when held in the compressed condition by the sheath.

A closely related aspect of the disclosure provides a retrieval catheter for retrieving a deployed prosthetic valve (or other cardiovascular implant), especially but not exclusively a valve according to any of the preceding aspects.

The retrieval catheter may comprise:
a catcher for catching on a retrieval feature of the prosthetic valve; and
at least one sheath;
the sheath and the catcher being displaceable axially with respect to one another between a first condition in which the catcher extends axially beyond a mouth of the sheath for catching on a prosthetic valve, and a second condition in which the catcher resides within the sheath for retrieving the prosthetic valve with respect to the sheath.

In some embodiments, the retrieval catheter comprises a first sheath and a second sheath disposed one within another and axially slidable with respect to each other and with respect to the catcher. The first (e.g. inner) sheath may surround closely an elongate member for supporting the catcher. The second (e.g. outer) sheath may surround the first sheath, and have a diameter for containing, at least partly, and in use after capture, the prosthetic valve in an at least partially collapsed condition.

In use, the catcher may be extended with respect to the first and second sheaths to facilitate engaging the retrieval feature of the prosthetic valve that is to be retrieved. The first sheath may be extended towards the catcher and prosthesis to provide a support for a counter force when the catcher is tensioned with respect to the first sheath. This action may secure the catcher to the prosthetic valve and/or provide an initial tensioning action for at least partly drawing a portion of the prosthetic valve into a collapsed condition (optionally while not entering the first sheath). The first sheath may serve to restrain the prosthesis against axial movement. Subsequently, relative movement between the second sheath and the combination of the first sheath and catcher, may draw the prosthetic valve progressively into a collapsed condition in the second sheath, and/or move the second sheath progressively over the prosthetic valve.

In some embodiments, the catcher may change configuration between at least two of: a stowed condition for introduction; an open condition for catching on to the retrieval feature of the prosthetic valve; and a closed condition for capturing the retrieval feature with respect to the catcher. The stowed condition may be substantially the same as the closed position, or these positions may be different from one another.

In some embodiments, the catcher may comprise a single element, for example, a hook or barb for catching on the retrieval feature of the prosthetic valve. In alternative embodiments, the catcher may comprise a plurality elements, for example a plurality of jaws configured (i) to open (e.g. diverge) when the catcher is extended with respect to the first sheath, and (ii) to close (e.g. narrow) when the catcher is withdrawn relative to the first sheath. Such plural jaws may be easier to catch on to a prosthetic valve than a single element. The catcher could also use a lasso element to catch on the retrieval feature.

In a closely related aspect, the present disclosure provides a prosthetic cardiovascular valve (prosthesis), comprising:
a stent configured to be collapsible (or collapsed) to a collapsed condition for delivery to an implantation site by catheter, and expandable to an expanded configuration for implantation; and
a valve component mounted at least partly within the stent component, for defining a one-way valve for regulating blood flow through the prosthesis.

The cardiovascular valve may, in some embodiments, be a pulmonary vein valve for implantation within a pulmonary vein, for example, at or close to an ostium or the left atrium.

For such a pulmonary vein valve, the outer diameter of the stent in the expanded condition may optionally be at least 10mm, optionally at least 11mm, optionally at least 12mm, optionally at least 13mm, optionally at least 14mm, optionally at least 15mm, optionally at least 16mm. Additionally or alternatively, the outer diameter of the stent in the expanded condition may optionally be less than 24mm, optionally less than 23mm, optionally less than 22mm, optionally less than 21mm, optionally less than 20mm.

Additionally or alternatively, the length of at least a trunk portion of the stent (e.g. a portion housing the valve component and/or providing the main structural tube of the stent) in the expanded condition may optionally be less than 25mm, optionally less than 24mm, optionally less than 23mm, optionally less than 22mm, optionally less than 21mm, optionally less than 20mm, optionally less than 19mm, optionally less than about 18mm, optionally less than about 17mm, optionally less than about 16mm, optionally less than about 15mm. A relatively short stent may reduce risk of the stent occluding tributaries of the pulmonary vein. A short stent also facilitates articulation of the delivery catheter for delivering the prosthetic valve to the pulmonary vein, for example, via transseptal approach through the atria. In one example, the length of the trunk portion may generally be in the range of 15 to 20mm, optionally about 18mm.

In some embodiments, the valve component may comprise at least one flexible leaflet optionally mounted on or carried by a flexible skirt (also called a "conduit" herein). Optionally, the number of leaflets may be two or three leaflets, as desired. If provided, the flexible skirt/conduit may facilitate mounting of the leaflet(s) to the stent, and/or promote sealing to prevent leakage around the leaflets. The leaflet(s) and the skirt may be made of the same or different material as each other. Suitable materials include biological tissue (for example, pericardial tissue such as porcine or bovine pericardial tissue), or synthetic fabrics, sheets or composites (for example, of PET or polyurethane).

In some embodiments, the leaflets and/or the skirt (if provided) may comprise flexible extension tabs extending outside the stent, for providing independently flexible seal segments to promote sealing against surrounding tissue of the vessel wall.

In some embodiments, the skirt (if provided) may comprise plural apertures for admitting blood flow between the interior and the exterior of the stent. Such apertures may reduce risk of occluding tributaries to the pulmonary vein even if in use the trunk portion of the stent carrying the skirt happens to directly overlap a tributary.

In some embodiments, the valve component may comprise wash-through passages permitting some blood to bypass the leaflets, thereby to avoid thrombus formation in the region of the wash-through apertures. The wash-through apertures may, for example, be arranged in a cusp region, or close to a cusp edge, of a leaflet of the valve component.

In some embodiments, the stent may comprise a single row of closed cells, for example, between four and eight cells, optionally six cells. The cells may have a generally diamond shape (at least in the expanded condition). Such an arrangement may balance the dimensions of the stent with the desired anchoring force for a pulmonary vein.

Additionally or alternatively to any of the foregoing, a hierarchical summary of certain aspects, features, ideas and embodiments, is also provided:
Aspect 1: A pulmonary vein prosthesis, comprising:
   a stent component, and
   a valve component;
   the stent component and the valve component being configurable in a collapsed configuration for delivery by catheter to a pulmonary vein implantation site, and an expanded configuration for implantation in a pulmonary vein.
Aspect 2: The prosthesis of aspect 1, wherein the stent component is of self-expanding material, the prosthesis further comprising a retrieval feature of the prosthesis for facilitating recollapsing and retrieval of the prosthesis from the implantation site, by means of a retrieval catheter, after implantation.
Aspect 3: The prosthesis of aspect 1 or 2, wherein the stent component is of self-expanding material, the prosthesis further comprising a flexible filament carried by the stent component, the flexible filament being permanently captive to the stent component.
Aspect 4: The prosthesis of aspect 3, wherein the flexible filament provides a retrieval feature of the prosthesis for facilitating recollapsing and retrieval of the prosthesis from the implantation site, by means of a retrieval catheter, after implantation.
Aspect 5: The prosthesis of aspect 4, wherein the flexible filament is configured such that application of external tension to the filament tends to draw a portion of the prosthesis radially inwardly, to facilitate recollapsing of the prosthesis.
Aspect 6: The prosthesis of aspect 3, 4 or 5, wherein the flexible filament serves to control, by means of a delivery catheter actuator coupled to the flexible filament, expansion of the prosthesis from the collapsed configuration towards the expanded configuration and/or collapsing of the prosthesis from the expanded configuration towards the collapsed configuration.
Aspect 7: The prosthesis of aspect 3 or any aspect dependent thereon, wherein the filament extends through apertures of the stent component.
Aspect 8: The prosthesis of aspect 2 or 3, or any aspect dependent on aspect 2 or 3, wherein the filament or retrieval feature is arranged at or towards an end of the stent component.
Aspect 9: The prosthesis of aspect 3 or any aspect dependent thereon, wherein the filament comprises at least one exposed filament portion.
Aspect 10: The prosthesis of aspect 3 or any aspect dependent thereon, wherein the stent component comprises a plurality of attachment elements for removable engagement with a stent holder, the attachment elements carrying the flexible filament.
Aspect 11: The prosthesis of any preceding aspect, wherein the stent component comprises a trunk portion comprising a single row of cells.
Aspect 12: The prosthesis of any preceding aspect, wherein the stent component comprises a trunk portion having a length of not more than 25mm.
Aspect 13: The prosthesis of any preceding aspect, wherein the stent component further comprises a plurality of contact projections extending from a trunk portion of the stent component towards an inlet end of the stent component.
Aspect 14: The prosthesis of any preceding aspect, wherein the valve component comprises a plurality of coapting leaflets, each leaflet comprising a mobile coaptation region and a cusp region opposite the coaptation region, wherein at least one opening is provided adjacent to a cusp region to permit washing-through of blood to reduce thrombosis formation.
Aspect 15: The prosthesis of any preceding aspect, wherein the valve component comprises a plurality of coapting leaflets, each leaflet comprising a mobile coaptation region and a cusp region opposite to the coaptation region, wherein at least a portion of the cusp region extends beyond the stent component to provide at least one seal extension, optionally at least two seal extensions, for sealing against a vessel wall.
Aspect 16: A transcatheter cardiovascular stent-valve prosthesis, optionally according to any preceding aspect, comprising:
   a stent component made of self-expanding material, and
   a valve component;
   the stent component and the valve component being configurable in a collapsed configuration for delivery by catheter to a cardiovascular implantation site, and an expanded configuration for implantation in a cardiovascular system;
   the prosthesis further comprising a flexible filament carried by the stent component and being permanently captive to the stent component, the flexible filament including at least one portion exposed for engagement by a manipulating device for applying tension to the flexible filament, the flexible filament being engaged with the stent component such that a configuration of at least a portion of the stent component is controllable in response to the applied tension.
Aspect 17: The prosthesis of aspect 16, wherein the flexible filament has a shape, in at least one configuration of the stent component, selected as one or more of: at least partly circumferential with respect to a portion of the stent component; at least partly radial with respect to a portion of the stent component; a figure-of-eight shape with respect to the stent component.
Aspect 18: The prosthesis of aspect 16 or 17, wherein the flexible filament has a closed loop shape.
Aspect 19: The prosthesis of aspect 16, 17 or 18, wherein the flexible filament is disposed towards an end of the stent component, optionally towards an outflow end with respect to the valve component.
Aspect 20: The prosthesis of aspect 16, 17, 18 or 19, wherein the flexible filament comprises or carries a feature that can be gripped or caught, optionally a bead, to facilitate engagement of the filament by a manipulating device.
Aspect 21: The prosthesis of any of aspects 16 to 20, further comprising a second flexible filament, optionally a third flexible filament, arranged at respective different positions along the length of the stent component.
Aspect 22: A transcatheter cardiovascular stent-valve prosthesis, optionally according to any preceding aspect, comprising:
   a stent component made of self-expanding material, and
   a valve component;
   the stent component and the valve component being configurable in a collapsed configuration for delivery by catheter to a cardiovascular implantation site, and an expanded configuration for implantation in a cardiovascular system;
   the prosthesis further comprising:
      at least one attachment element for removable engagement with a stent holder of a delivery catheter; and
      a retrieval feature for facilitating recollapsing and retrieval of the prosthesis from the implantation site, by means of a retrieval catheter, after implantation.
Aspect 23: The prosthesis according to aspect 22, wherein the retrieval feature is provided at, or is carried by, the at least one attachment element.
Aspect 24: A retrieval catheter for retrieving a deployed cardiovascular prosthesis, optionally a prosthesis according to any preceding aspect, the retrieval catheter comprising:
   a catcher for catching on a retrieval feature of the prosthesis, and
   at least one sheath;
   the catcher and the sheath being displaceable axially with respect to one another between a fist condition in which the catcher extends axially beyond a mouth of the sheath for catching on a prosthetic valve, and a second condition in which the catcher resides within the sheath for retrieving the prosthetic valve with respect to the sheath.
Aspect 25: The catheter of aspect 24, wherein the catcher is configured to change configuration between at least two of:
   a stowed condition for introduction;
   an open condition for catching on to the retrieval feature of the prosthetic valve;
   a closed condition for capturing the retrieval feature with respect to the catcher.
Aspect 26: The catheter of aspect 25, wherein the stowed condition is one of: substantially the same as the closed condition; or different from the closed condition.
Aspect 27: The catheter of aspect 24, 25 or 26, wherein the catcher comprises at least one selected from: a hook; a barb; a snare; a lasso; a pair of jaws.
Aspect 28: The catheter of any of aspects 24 to 27, wherein the catheter comprises first and second sheaths disposed one within another and axially slidable with respect to each other and with respect to the catcher.
Aspect 29: A delivery catheter for implanting a cardiovascular prosthesis of a type comprising at least one flexible filament that is part of the prosthesis for controlling expansion and/or collapsing of at least a portion of the prosthesis, the prosthesis optionally of a type according to any of aspects 1 to 23, the delivery catheter comprising:
   at least one shaft having a prosthesis accommodation region at or towards a distal end, and
   a manipulation device extending from the proximal end to the distal end of the shaft, the manipulation device comprising an engagement element at the stent accommodation region for releasably engaging the flexible filament of the prosthesis, and an actuator for applying or relaxing tension in the flexible filament, thereby to control expansion and/or collapsing of at least a portion of the prosthesis.
Aspect 30: The delivery catheter of aspect 29, wherein the engagement element comprises a snare loop.
Aspect 31: The delivery catheter of aspect 29 or 30, comprising a plurality of engagement elements for engaging a respective plurality of filaments of the prosthesis.
Aspect 32: The delivery catheter of aspect 31, wherein the actuator is configured for actuating the plural engagement devices in unison.
Aspect 33: The delivery catheter of aspect 31, comprising a plurality of actuators for actuating the engagement elements individually.
Aspect 34: A combination of a catheter as defined in any of aspects 24 to 33 and a prosthesis as defined in any of aspects 1 to 23.
Aspect 35: A combination of:
   at least one prosthesis according to any of aspects 1 to 23 for implantation in at least one respective pulmonary vein; and
   a space-occupying member for implanting into a left atrium for reducing the reservoir volume of the left atrium between the pulmonary veins and a native mitral valve.

### Brief Description of the Drawings

Fig. 1 is a schematic section showing the left ventricle of a heart in systole.
Fig. 2a is a schematic partial posterior view of a left atrium, showing prosthetic valves positioned at the ostia of pulmonary veins.
Fig. 2b is a schematic partial cut-away anterior view of a left atrium corresponding to Fig. 2a, showing prosthetic valves positioned at the ostia of pulmonary veins.
Fig. 3 is a schematic partial anterior view of a left atrium similar to Fig. 2b showing the addition of an atrial volume reducer.
Fig. 4 is a schematic perspective view of a prosthetic pulmonary vein valve.
Fig. 5 is a schematic perspective view of a second example of pulmonary vein valve.
Fig. 6 is a schematic perspective view of a third example of pulmonary vein valve.
Fig. 7 is a schematic perspective view of a fourth example of pulmonary vein valve.
Fig. 8 is a schematic side view of a fifth example of pulmonary vein valve.
Fig. 9 is a schematic unrolled view of the valve of Fig. 8.
Fig. 10 is a schematic illustration of a use of a retrieval feature of the valve of Figs. 6, 8 and 9.
Fig. 11 is a schematic perspective view of a sixth example of pulmonary vein valve.
Fig. 12 is a schematic unrolled view of the valve of Fig. 11.
Fig. 13 is a schematic illustration of use of a retrieval feature of the valve of Figs. 11 and 12.
Fig. 14 is a schematic illustration of a modification of the stent of any of the examples described herein, and showing an attachment element with a collapsible retrieval feature.
Fig. 15 is a schematic illustration of a first version of collapsible retrieval feature detail of Fig. 14.
Fig. 16 is a schematic illustration of a second version of collapsible retrieval feature detail of Fig 14.
Fig. 17 is a schematic illustration of the attachment element with the retrieval feature in its collapsed condition.
Fig. 18 is a schematic illustration of a modification of the valve component of any of the valves described herein, providing additional paravalvular sealing.
Fig. 19 is a schematic illustration of a stent of a seventh example of pulmonary vein valve, shown in a rolled-out, as-cut, collapsed configuration.
Fig. 20 is a schematic illustration of the stent of Fig. 19 (rolled out) in an expanded configuration.
Fig. 21 is a schematic partial perspective view of a unit cell of the stent of Fig. 20.
Fig. 22 is a schematic perspective view of a distal end of a deliver catheter for the valve of Figs. 19-21.
Fig. 23 is an enlargement of the stent holder of Fig. 22.
Fig. 24 is a schematic illustration of a stent of an eighth example of pulmonary vein valve, shown in a rolled-out, as-cut, collapsed configuration.
Fig. 25 is a schematic illustration of the stent of Fig. 24 (rolled out) in an expanded configuration.
Fig. 26 is a schematic partial perspective view of a unit cell of the stent of Fig. 25.
Fig. 27 is a schematic illustration of a stent of a ninth example of pulmonary vein valve, shown in a rolled-out, expanded configuration.
Fig. 28 is a schematic perspective view showing engagement of the stent of Fig. 27 in a collapsed condition, with a stent-holder of a delivery catheter.
Fig. 29 is a schematic side perspective view of the stent holder from Fig. 28 in isolation.
Fig. 30 is a schematic perspective view of a tenth example of pulmonary vein valve, shown in the expanded condition.
Fig. 31 is a schematic perspective view of the attachment elements of the stent of Fig. 30 engaging, in the collapsed condition, a stent holder of the delivery catheter.
Fig. 32 is a schematic perspective view of the stent holder of Fig. 31 shown in isolation.
Fig. 33 is a schematic perspective view of a conduit for a valve component.
Figs. 34a and 34b are schematic side views of two alternative leaflet blanks in flat configuration.
Fig. 35 is a schematic perspective view showing a first step of valve component assembly.
Fig. 36 is a schematic perspective view showing a second step of valve component assembly.
Fig. 37 is a schematic perspective view showing a third step of valve component assembly.
Figs. 38a and 38b are schematic perspective views showing two alternative examples of a fourth step of valve component assembly.
Figs. 39a and 39b are schematic perspective views showing two alternative examples of a fifth step of valve component assembly for the two options of Figs. 38a and 38b, respectively.
Figs. 40a and 40b are partial schematic side views showing a first mounting step for mounting the valve component commissure to the stent, for the two leaflet designs of Figs. 34a and 34b, respectively.
Fig. 41 is a schematic cross-section along the line A-A of Fig. 40a.
Fig. 42 is a partial schematic side view showing completion of the mounting of the valve component commissure to the stent.
Fig. 43 is a schematic section along the line A-A of Fig. 42.
Figs. 44a and 44b are schematic rolled-out side views showing completion of assembly of the valve, for the alternative options of Figs. 39a and 39b, respectively.
Fig. 45 is a schematic view from one end of the stent of any of Figs. 19 to 32, showing a first configuration of retrieval filament.
Fig. 46 is a schematic view from one end of the stent of any of Figs. 19 to 32, showing a second configuration of retrieval filament.
Fig. 47 is a schematic illustration showing a third configuration of retrieval filament for the stents of any of Figs. 19 to 32 with reference to the apertures of the attachment elements.
Fig. 48 is a schematic section showing a distal end of a first example of retrieval catheter.
Fig. 49 is a schematic section showing a first step of using the catheter of Fig. 48.
Fig. 50 is a schematic section showing a second step of using the catheter of Fig. 48.
Fig. 51 is a schematic section showing a third step of using the catheter of Fig. 48.
Fig. 52 is a schematic section showing a fourth step of using the catheter of Fig. 48.
Fig. 53 is a schematic side view of a second example of retrieval catheter.
Fig. 54 is a schematic partial section showing a detail of the distal end of the catheter of Fig. 53 on an enlarged scale.
Fig. 55 is a schematic perspective view showing how the catcher depicted in Fig. 54 may engage an implant.
Fig. 56 is a schematic perspective view similar to Fig. 55, showing closing of the catcher.
Fig. 57 is a schematic side view showing a step of progressive collapsing of the implant into the sheath of the retrieval catheter.
Fig. 58 is a schematic side view similar to Fig. 57, showing completion of collapsing of the implant into the sheath of the retrieval catheter.
Fig. 59 is a schematic perspective view of a stent similar to that of Fig. 30 illustrating a further example of a flexible retrieval element.
Fig. 60 is a schematic enlarged view showing a detail of Fig. 59, in particular an arrangement of the flexible retrieval element passing through apertures of the stent.
Fig. 61 is a schematic side perspective view of the distal end of a further example of delivery catheter.
Fig. 62 is a schematic perspective view showing advancement of a snare.
Fig. 63 is a schematic perspective view showing use of the snare in the delivery catheter of Fig. 61 for controlling expansion and collapsing of a prosthesis.
Fig. 64 is a schematic perspective view of the distal end of a further example of delivery catheter.
Fig. 65 is a schematic view similar to Fig. 64, illustrating collapsing of the prosthesis.
Fig. 66 is a schematic view similar to Fig. 64, illustrating expansion of the prosthesis.
Fig. 67 is a schematic perspective view of the distal end of a further example of delivery catheter.
Fig. 68 is a schematic view showing a detail of Fig. 67 on an enlarged scale.
Fig. 69 is a schematic side view showing a prosthesis attached to the catheter of Fig. 67.
Fig. 70 is a schematic perspective view similar to Fig. 69.
Fig. 71 is a schematic distal end view of the arrangement of Figs. 69 and 70, except that two of the three flexible filaments are omitted to avoid obscuring the drawing.
Fig. 72 is a schematic side view similar to Fig. 69, but illustrating an alternative example of controlling expansion and collapsing of the prosthesis.
Fig. 73 is a schematic perspective view showing a further example of a stent of an implantable prosthesis similar to Fig. 30, but with modified contact projections and a modified arrangement of attachment elements.
Fig. 74 is a schematic side view, on an enlarged scale, of a detail of a contact projection of the stent of Fig. 73.
Fig. 75 is a schematic view of the distal end of a delivery catheter into which the stent of Fig. 73 has been loaded in its collapsed condition.
Fig. 76 is a schematic view of the distal end of the delivery catheter of Fig. 75, with the stent in a partially unsheathed condition.
Fig. 77 is a schematic illustration indicating respective example diameters of a blood vessel and a stent, side by side.
Fig. 78 is a schematic illustration showing the stent implanted into the blood vessel of Fig. 77.
Fig. 79 is a schematic perspective illustration, on an enlarged scale, of a further example of antimigration element.
Fig. 80 is a schematic top view showing collapsing of the stent of Fig. 73 by a retrieval catheter, in a manner similar to Fig. 57.

### Detailed Description of Preferred Embodiments

Non-limiting embodiments of the disclosure are now described, by way of example only, with reference to the drawings. In the drawings, like reference numerals are used to denote the same or equivalent features unless described to the contrary.

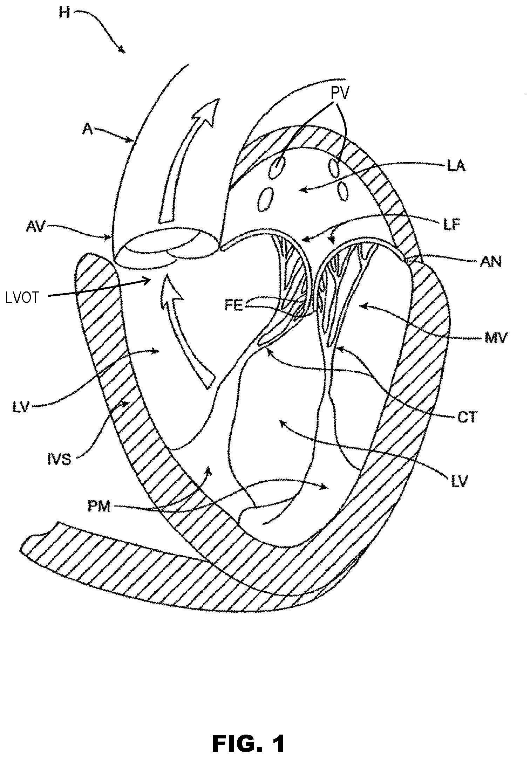

Fig. 1 shows a left ventricle LV and left atrium LA of a heart H. Oxygenated blood from the lungs enters the left atrium LA via pulmonary veins PV. Four pulmonary veins are illustrated, which is the typical number, although some people have five. The left atrium LA acts as a buffer or reservoir for the left ventricle LV. During cardiac diastole (not depicted specifically in Fig. 1), the left ventricle LV fills with this blood from the left atrium LA, via the mitral valve MV, by a sequence of passive filling followed by atrial contraction.

During cardiac systole (depicted in Fig. 1), the left ventricle LV contracts to pump the blood through the left ventricle outflow tract (LVOT) and aortic valve AV into the aorta A as depicted by the blow flow arrows. A healthy mitral valve MV prevents reverse flow to the left atrium LA and back into the pulmonary veins PV when pressure in the left ventricle is higher than that in the left atrium LA. The mitral valve MV comprises a pair of leaflets having free edges FE at an outlet end, which coapt as illustrated in Fig 1. The opposite ends of the leaflets LF are attached to the surrounding heart structure via an annular region of tissue referred to as the annulus AN. The annulus AN corresponds to an inlet end of the mitral valve. The free edges FE of the leaflets LF are secured to the lower portions of the left ventricle LV through chordae tendineae CT (referred to hereinafter as "chordae") which include a plurality of branching tendons secured over the lower surfaces of each of the valve leaflets LF. The chordae CT in turn, are attached to the papillary muscles PM, which extend upwardly from the lower wall of the left ventricle and interventricular septum IVS.

A number of structural defects and diseases can affect the function of the mitral valve, leading to mitral valve regurgitation. For example, ruptured chordae can allow a valve leaflet to prolapse due to inadequate tension retaining the leaflet from the ventricle side. An increase in the size of the mitral annulus, due to structural defects or illness, can also result in inadequate leaflet coaptation or permanent separation. Ischemic heart disease can impair functioning of the papillary muscles PM to draw the leaflets to coapt as part of the cardiac cycle. Damage to the leaflets themselves can also result in leakage through the valve. Infective endocarditis could lead to the damage of valve leaflets and severe acute mitral regurgitation.

Not only does a dysfunctional mitral valve reduce the efficiency with which the heart can circulate oxygenated blood to the body, it also subjects the left atrium LA and the pulmonary veins PV to high systolic blood pressure, which does not occur with a healthy mitral valve. This can lead to concomitant enlargement of the atrium, dilation of the pulmonary veins, and pulmonary venous and arterial hypertension, which reduces blood flow through the lungs and hence the amount of oxygenated blood available. Accumulation of the blood in the pulmonary veins and arteries can result in pulmonary oedema, which is a severe life threatening complication.

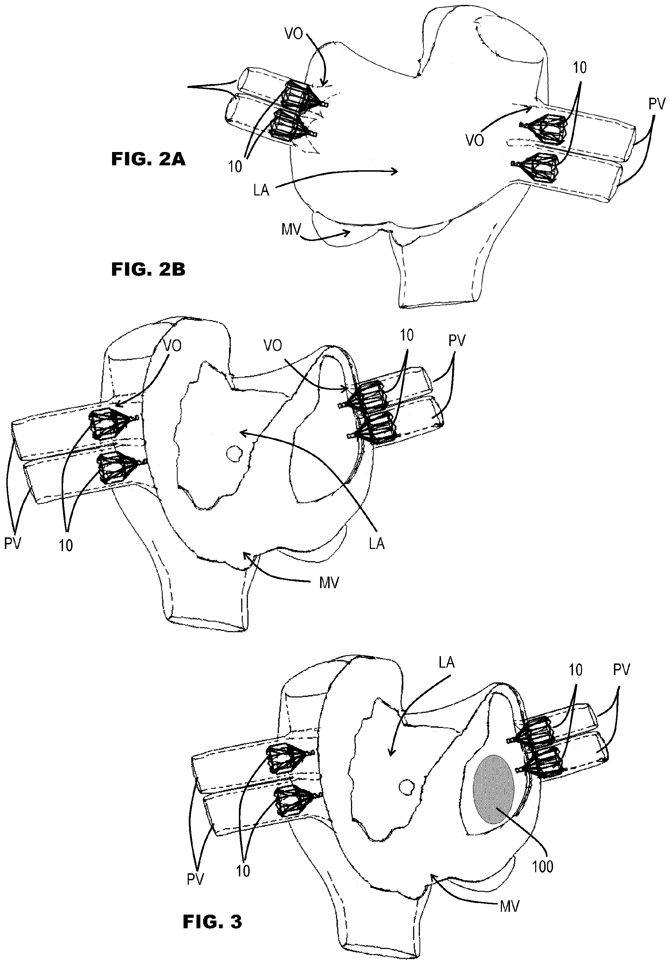

Referring to Fig. 2, one aspect of the present disclosure provides use of at least one, preferably two, more preferably three, and most preferably at least four (optionally five), prosthetic valves 10 configured for implantation in the pulmonary veins PV. Optionally, the prosthetic valves are positioned at or close to the vein ostia (VO) where the vein enters the atrium (LA). The prosthetic valve(s) 10 may function upstream of the mitral valve MV (depicted without detail in Fig. 2) to reinforce the mitral valve function and/or to mitigate effects of mitral valve dysfunction. The prosthetic valve(s) 10 may close to prevent undesired back-flow of blood into the pulmonary veins PV during ventricular systole, and reduce exposure of the pulmonary veins PV to high ventricular systolic pressure. The number of prosthetic valves 10 may vary depending on the characteristics of the patient and/or the preferences of the practitioner. While four (or five, as appropriate) prosthetic valves 10 may provide most comprehensive protection against blood back-flow and systolic pressure, in some cases the number of valves may instead be three, two or even a single valve.

Implementation of a pulmonary vein valve requires careful design to suit the characteristics of the pulmonary vein. The pulmonary vein does not support any native valve, and so does not have any natively reinforced annulus tissue. Instead, the pulmonary vein wall is somewhat elastic. Also, the vein wall is relatively thin so less adapted to withstand substantial anchoring forces or be engaged by aggressive anchoring profiles. A further important consideration is that the pulmonary vein comprises multiple tributary vessels leading from the lung into the main vein vessel, even close to the ostium. Avoiding occlusion of these tributary vessels is important for maintaining good perfusion of blood through the lung tissue, and free flow of oxygenated blood from the lungs to the heart for circulation to support bodily function.

Referring to Fig. 3, in order to reduce the regurgitant volume between the mitral valve MV and the prosthetic valves 10, one or more space-occupying members (or volume-reducers) 100 may optionally be implanted within the left atrium LA. The member 100 has a three-dimensional shape, for example, spherical, frusto-spherical, or other bulbous shape. By occupying some of the cavity space of the atrium, the member 100 can further reduce the effects of there being reservoir volume between the dysfunctional mitral valve MV, and the prosthetic valves 10 that ultimately prevent backflow. By decreasing the size of this reservoir, the volume reducers will decrease the mitral regurgitant volume and increase the desirable systemic (transaortic) forward ejected LV volume. The member 100 may itself be expandable and/or collapsible, and be implantable and/or retrievable using a catheter. For example, the member 100 may comprise a balloon or bladder that is distensible by means of an inflation fluid (e.g. a liquid, such as saline, patient's blood, contrast medium, etc) or gas (e.g. a gas CO2, helium, argon, xenon, etc). Additionally or alternatively, the member 100 may comprise or be made of self-expandable foam.

The member 100 may be fixed to the wall of the left atrium by any suitable means, for example, by being anchored at a left atrial appendage of the atrium, attached to the inter-atrial septum or to the prosthetic valves implanted to the pulmonary veins.

The member 100 may be used whether or not the atrium (LA) has been enlarged as a result of the mitral valve dysfunction. However, in the case of an enlarged atrium (LA), the member 100 may be especially useful in compensating for (at least partly, wholly or even overcompensating) the volume increase of the atrial cavity.

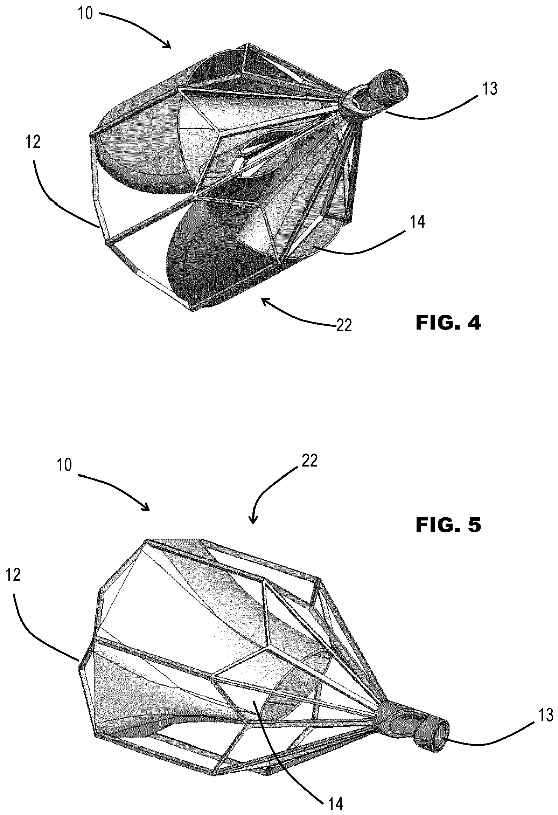

Figs. 4-44 show example prosthetic valves 10, or details thereof. The valve designs are especially suitable for use in the pulmonary vein(s), but may also be used anywhere in the cardiovascular system of a patient, for example, to replace a cardiac valve or a venous valve.

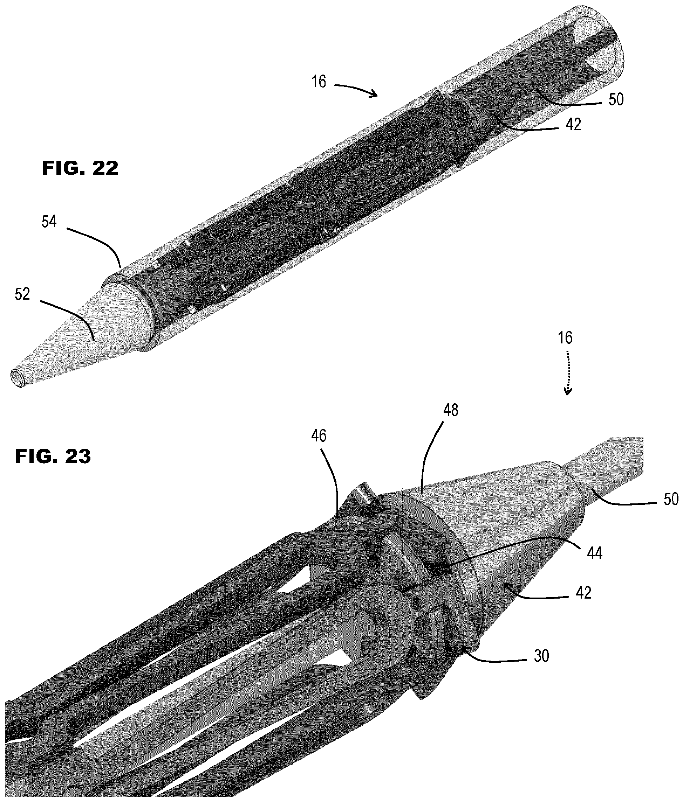

The prosthetic valve 10 comprises a stent 12 and a valve component 14. Some of the drawings may omit one or more details or components to avoid clutter, but it is to be understood that both components 10 and 12 may be present in all embodiments. The stent 12 and valve component 14 are collapsible (or collapsed) to a collapsed condition for delivery to the implantation site by a delivery catheter 16 (Figs. 22 and 61-70). The stent 12 and valve component 14 are expandable from the collapsed condition to an expanded condition for implantation. The stent 12 may be at least partly self-expanding from the collapsed condition towards the expanded condition, and may be made of a suitable shape memory material, for example, a shape memory alloy. A suitable shape memory alloy may, for example, be an alloy comprising nickel and titanium, for example, nitinol. In some embodiments, and whether or not the stent is at least partly self-expanding, the stent may be configured to be forcibly expanded (e.g. by means of a dilation balloon or by application of mechanical forces tending to expand the stent). In the illustrated embodiment, the stent 12 is self-expanding, which facilitates partial expansion, recapture and retrieval, as described later below. The stent 12 may have a generally tubular and/or closed loop form. The stent 12 may, for example, be cut (e.g. laser cut) from a tube of the material.

Various different designs of stent 12 and valve component 14 are envisaged and illustrated in Figs. 4-44. The stent 12 may have a body or trunk portion 22 supporting the valve component 14 and/or providing the structural tube of the stent. The stent 12 may serve to support the valve component 14, and anchor the prosthesis in position when implanted.

For pulmonary vein applications, it may be preferred that the axial length of the trunk portion 22 may, in the expanded condition, optionally be less than 25mm, optionally less than 24mm, optionally less than 23mm, optionally less than 22mm, optionally less than 21mm, optionally less than 20mm, optionally less than 19mm, optionally less than about 18mm, optionally less than about 17mm, optionally less than about 16mm, optionally less than about 15mm. Such a relatively short stent may reduce risk of the stent occluding tributaries of the pulmonary vein. A short stent also facilitates articulation of the delivery catheter for delivering the prosthetic valve to the pulmonary vein, for example, via transseptal approach through the atria. In some examples, the length of the trunk portion 22 may generally be in the range of 15 to 20mm, optionally about 18mm.

Additionally or alternatively, for a pulmonary vein valve, the outer diameter of the stent in the expanded condition may optionally be at least 10mm, optionally at least 11mm, optionally at least 12mm, optionally at least 13mm, optionally at least 14mm, optionally at least 15mm, optionally at least 16mm. Additionally or alternatively, the outer diameter of the stent in the expanded condition may optionally be less than 24mm, optionally less than 23mm, optionally less than 22mm, optionally less than 21mm, optionally less than 20mm.

Figs. 4 and 5 illustrate a stent 12 generally in the form of a cage-like structure which tapers at one end (e.g. the outflow end) to a hub 13. The hub 13 may serve as a manipulation element by which the stent 12 may be manipulated (e.g. held or grabbed) by a catheter (i) for deployment (from the collapsed configuration to the expanded configuration) and/or (ii) for retrieval (from the expanded configuration to the collapsed configuration).

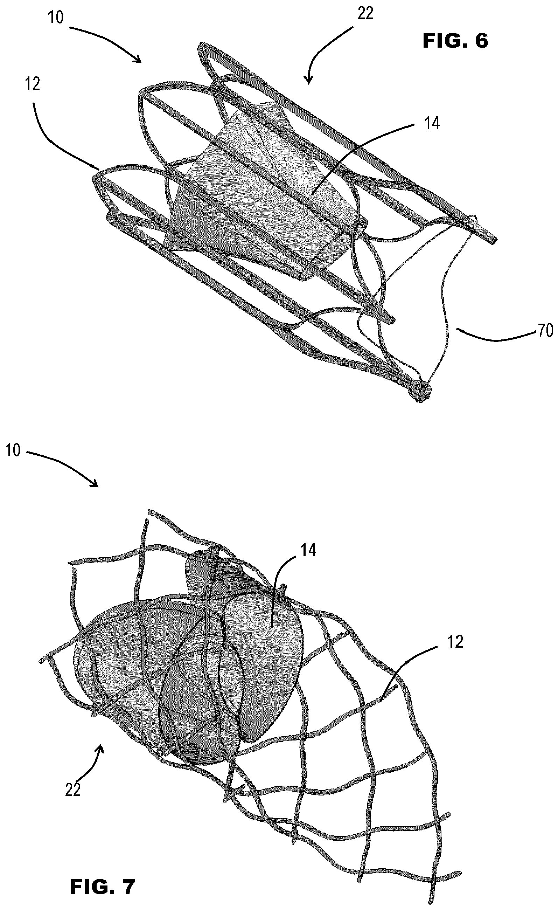

Fig. 6 illustrates a stent 12 generally in the form of a tubular cage supporting at one end a retrieval feature in the form of a filament 70 (described in more detail later below).

Fig. 7 illustrates a stent 12 generally in the form of a tubular mesh or braid. The mesh/braid may optionally be beveled having a circumferential asymmetrical extension at one end (e.g. an outflow end) to adapt to local anatomy and/or to provide structure that may be hooked or grabbed by a retrieval catheter and easily pulled into the retrieval sheath.

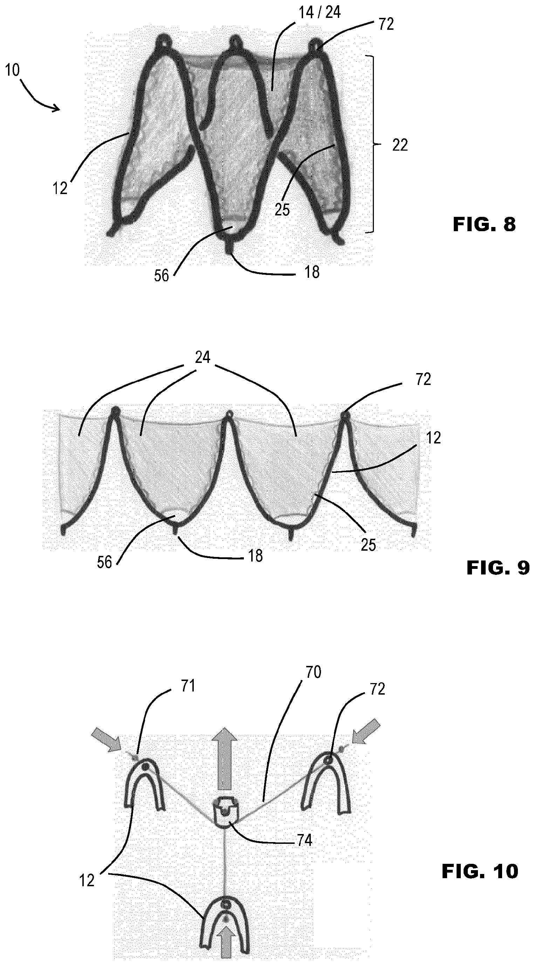

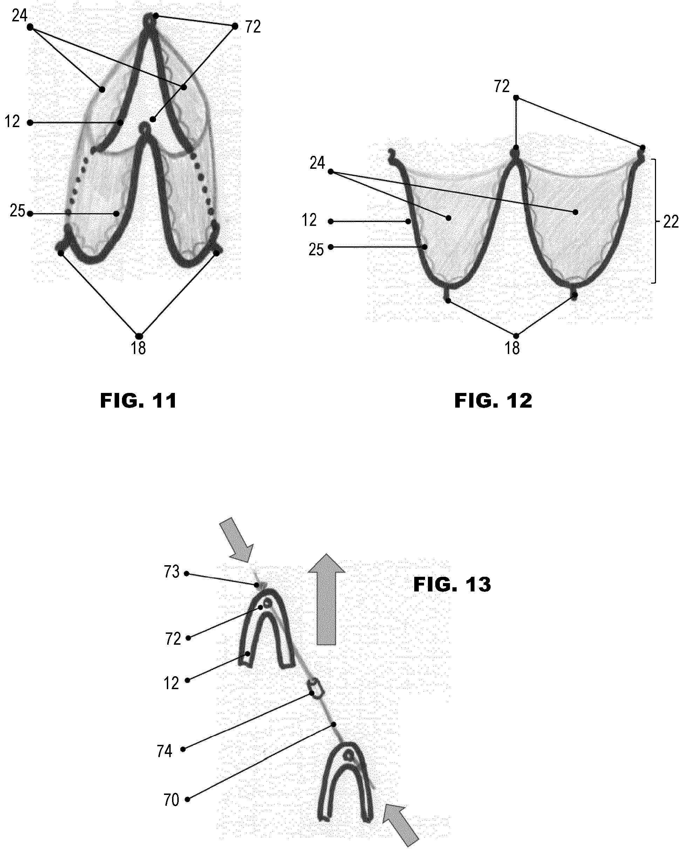

Figs. 8-18 illustrate a stent 12 comprising a trunk portion 22 in the form of a generally sinuous strut or wire extending in the circumferential direction and defining peaks and troughs. For example, in Figs. 8-9, the stent may have three peaks and three troughs (optionally matching a cusp and commissure configuration of a tri-leaflet valve). In Figs. 11-12, the stent 12 may have two peaks and two troughs (optionally matching a cusp and commissure configuration of a bi-leaflet valve).

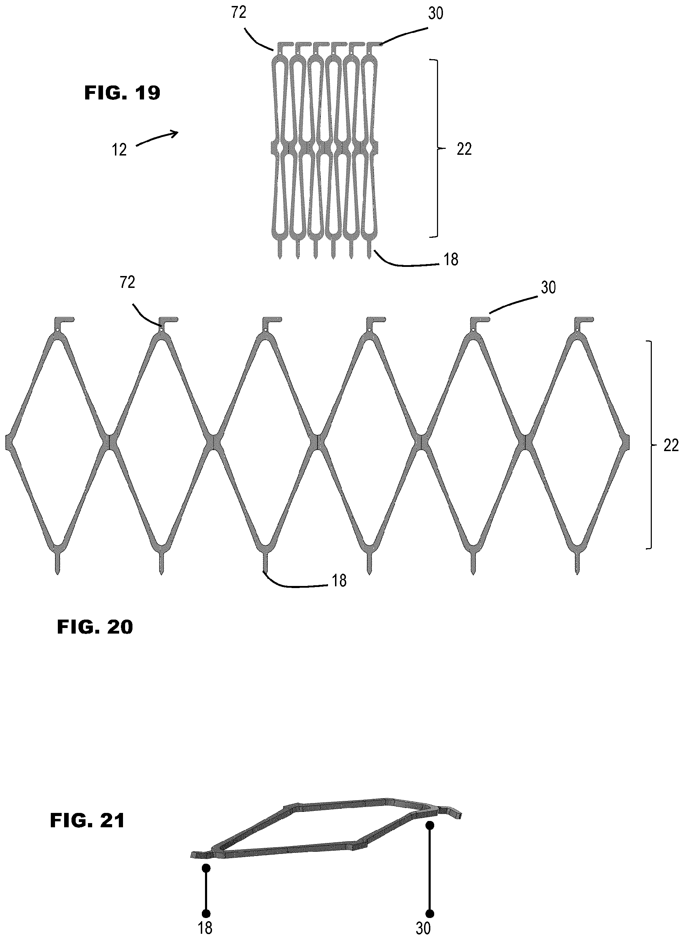

Figs. 19-22, 24-27, 30 and 44a-b illustrate a stent 12 comprising a trunk portion 22 in the form of generally diamond-shaped cells defining a tubular lattice. The cells may be arranged in a single circumferential row. For example, selected apexes of the cells may correspond to the positions of peaks and/or troughs of the sinuous arrangement of Figs. 8-9. In the illustrated example, three apexes (every second in sequence) correspond to the positions of peaks of the sinuous arrangement.

While a number of specific designs of trunk portion 22 have been described, the disclosure is not limited to these. The trunk portion 22 may also have alternative cell, or net-like, or zig-zag configurations that permit the stent to be collapsed for delivery, and expanded (or expandable) at implantation.

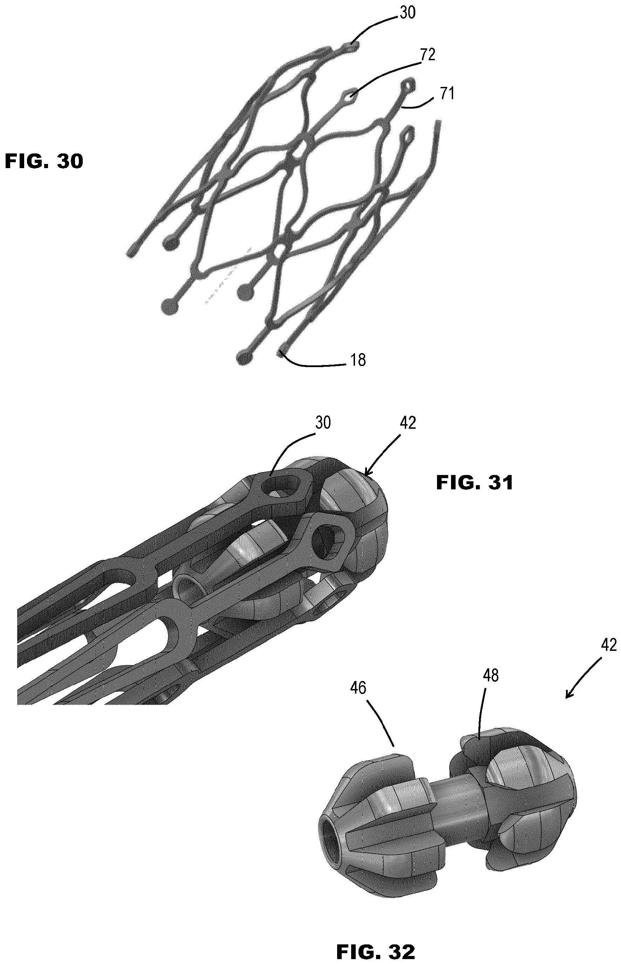

In some embodiments (and whether or not shown explicitly in the drawings), one end of the stent 12 (for example, an inflow end) may optionally comprise a plurality of contact projections 18. The contact projections 18 may extend generally axially (e.g. Fig. 30), or they may diverge at least somewhat to define a flared profile (Figs. 8, 11). The contact projections 18 of Fig. 30 may also be configured to diverge at least somewhat if desired.

The contact projections 18 engage the vessel wall in the implanted, expanded configuration. In some embodiments, the contact projections 18 may serve to reinforce anchoring by resisting displacement of the stent 12. Additionally or alternatively, in some embodiments, the contact projections 18 may serve to stabilize the stent during deployment and expansion. The contact projections 18 may be the first portions of the stent 12 to contact the vessel wall during progressive deployment, and may provide stable points of contact around the periphery to reduce any risk of the relatively short stent 12 tilting undesirably with respect to the vessel axis.

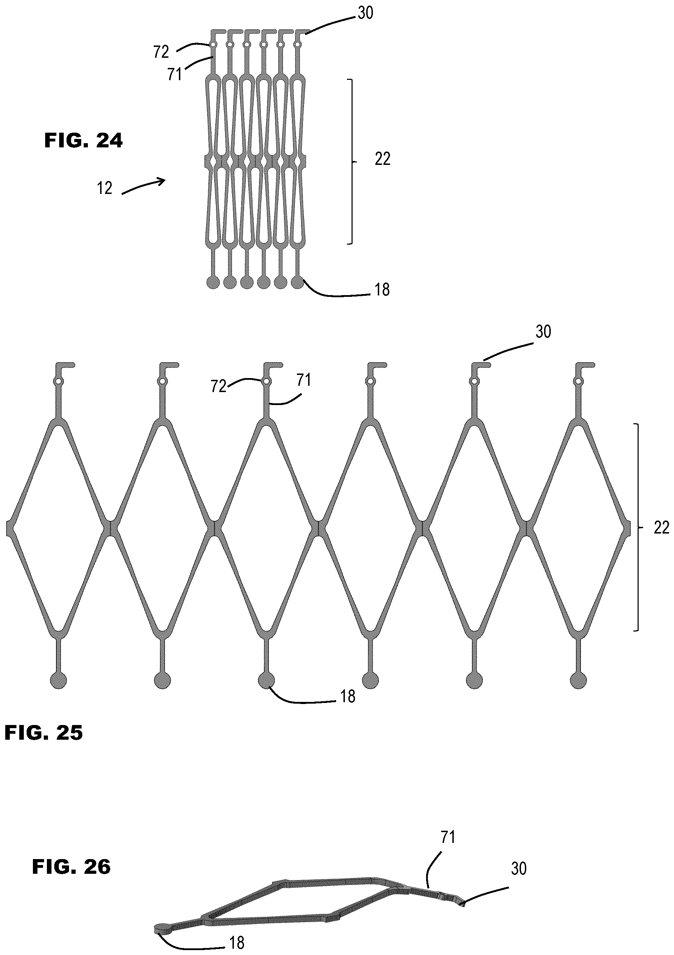

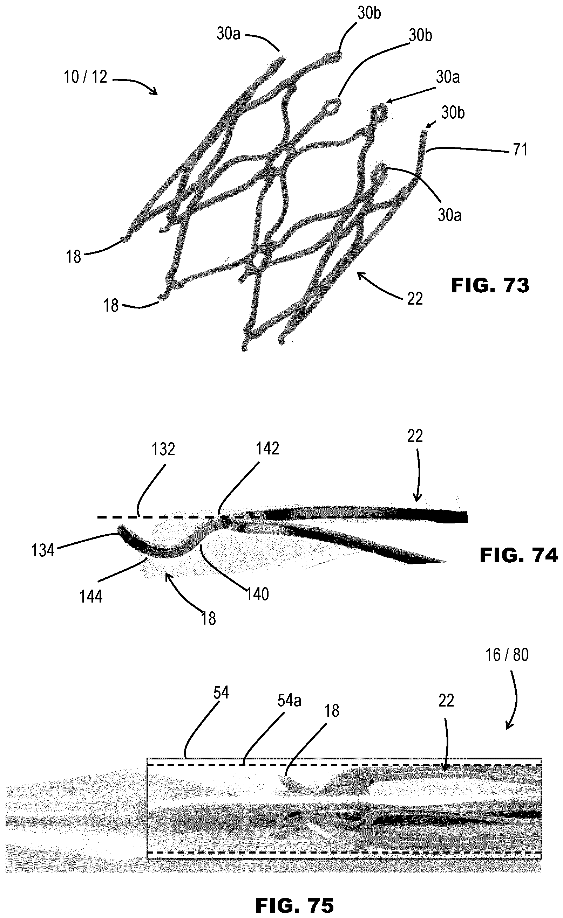

In some examples (Fig. 24-27, 30, 44a-b), the contact projections 18 may have a generally atraumatic shape. An atraumatic shape may reduce the risk of perforating or otherwise damaging the relatively thin elastic vessel wall of the pulmonary vein. In the illustrated examples, the contact projections 18 have a generally circular pad-like shape. The outer surface of those pad-like contact projections can be roughened, embossed, grained or covered by elements increasing friction between the pad and the vein wall (e.g.,. micropins, spikes, hooks, projections, sawtooth profiles, etc). In other examples, the contact projections 18 may be configured to lodge more forcefully within the surrounding vessel wall. For example, Figs. 8-9, 11-12, and 19-21 depict contact projections 18 in the form of barbs or spikes. Figs 73-79 (described later below) illustrate further examples of contact projections 18 in the form of anti-migration elements projecting axially, but not radially, with respect to the trunk portion of the stent.

Anchoring of the prosthesis 10 is an important consideration for the pulmonary vein. Depending on the specific embodiment, considerations include one or more of:
(i) resisting migration when axial forces acting on the prosthesis are at their highest, namely when the valve component 14 closes to prevent reverse blood flow, and the stent 12 has to bear the force of the blood pressing on the prosthesis in the direction towards the inflow; and/or
(ii) resisting migration in a forward direction, e.g. towards the heart for a pulmonary vein valve, as a result of anatomy and forward blood flow;
(iii) ability to retrieve the prosthesis after implantation.

In some embodiments, anchoring by the trunk portion of the stent 12 and/or the contact projections 18 may be sufficient. If desired, additional anchoring may be provided by increasing the friction of the contact projections 18 as described above. The increased friction is such that collapsing and retrieval of the prosthesis after implantation is not substantially impeded. Further reference is made to the antimigration elements described below with respect to Figs. 73-79 that may optionally be implemented to enhance anchoring.

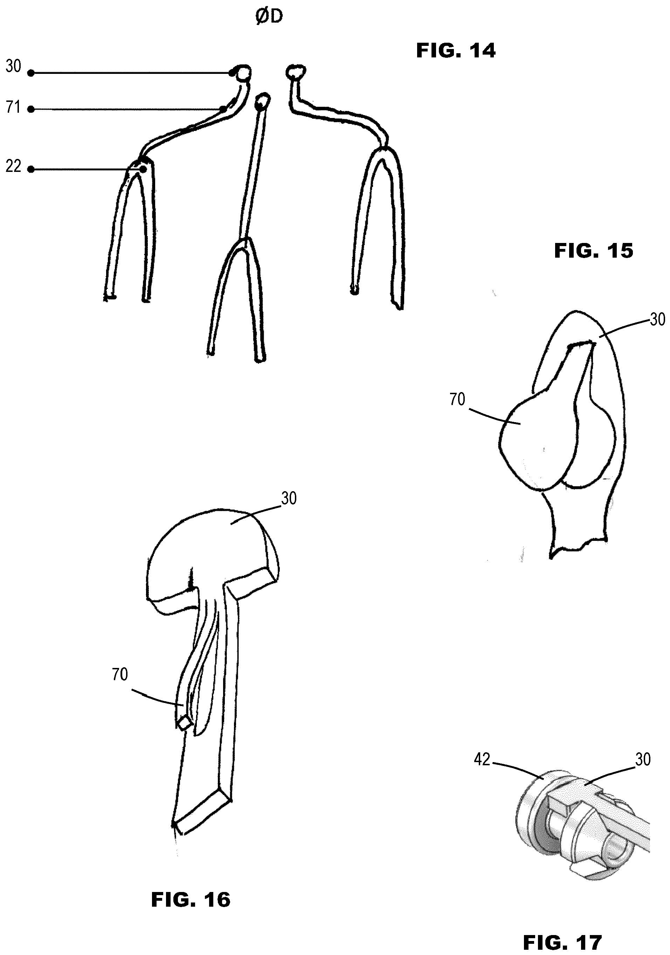

Additionally or alternatively to the above and whether or not shown explicitly in the drawings, one end of the stent (for example, an outflow end) may comprise a plurality of attachment elements 30 (Figs. 14-17, 19-21, 23-28, 31-32, 73 and 80) configured for engaging a stent holder 42 of a delivery catheter used to deliver the prosthesis 10. The attachment elements 30 may serve to prevent axial displacement of the prosthesis with respect to the stent holder 42 of the delivery catheter, at least in one axial direction, while the attachment elements 30 remain engaged with the stent holder 42. In the case of a self-expanding prosthesis, the attachment elements 30 may prevent the prosthesis from tending to pop-out prematurely from the delivery catheter during the deployment procedure.

The attachment elements 30 may have the form of one or more of: projections (e.g. T-shaped, or L-shaped, or mushroom-head shaped, or diamond-shaped), hooks, crotchets, paddles, apertures (e.g. round, oval, or C-shaped).

Figs. 19-26 illustrate L-shaped attachment elements 30 that may engage a stent holder 42 (Fig. 23) having a circumferential groove 44 defined between a segmented front wall 46 and a rear wall 48. In Fig. 23, for reasons of drawing limitations, the base of the L-shape is shown having a tangential direction, although it will be appreciated that if the stent is cut from tubular material, the base will naturally be curved in the circumferential direction to fit within the circumferential groove 44.

Referring to Fig. 22, the delivery catheter comprises an inner shaft or tube 50 carrying the stent holder 42 and an atraumatic tip 52. The delivery catheter further comprises a constraining sheath 54 for constraining the stent within the sheath, in its collapsed configuration for delivery. In use, during deployment, the constraining sheath 54 is withdrawn progressively in the axial direction, allowing the stent 12 to expand (e.g. self-expand) from the end closest to the atraumatic tip 52, to a partially expanded configuration. During this initial expansion, the attachment elements 30 remain constrained by the sheath 54, and engaged to the stent holder 42. The L-shaped attachment elements prevent any axial slippage of the stent-valve, and also prevent it from popping out prematurely from the sheath 54. In this example, the configuration of the stent holder and the attachment elements restrains the stent in both axial directions. Once the sheath 54 has been withdrawn past the stent holder 42, the attachment elements 20 are free to disengage from the stent holder 42 (e.g. due to self-expansion of the stent 12 to the fully expanded configuration).

In the example shown in Figs. 19-23, the attachment elements 30 are adjacent to the trunk portion 22. In alternative examples of Figs. 24-32, the attachment elements 30 are spaced axially from the trunk portion. For example, the attachment elements 30 may comprise or be supported by struts or stalks 71. Such an arrangement may permit the trunk portion 22 to expand more fully in the partially expanded configuration, to allow the position of the stent to be observed more easily and accurately using medical imaging (e.g. X-ray imaging) before the deployment is complete, and/or to allow the functionality of the valve to be verified via medical imaging (e.g. echography or fluoroscopy) before the deployment is complete.

Similarly, in the example shown in Figs. 19-23, the contact projections 18 are adjacent to the trunk portion 22. In the alternative examples of Figs. 24-32, the contact projections 18 are spaced axially from the trunk portion 22. This may permit a more complete and stable contact of the projections 18 with the vessel wall before unsheathing of the trunk portion 22, in order to stabilize the prosthesis for unsheathing of the trunk portion 22.

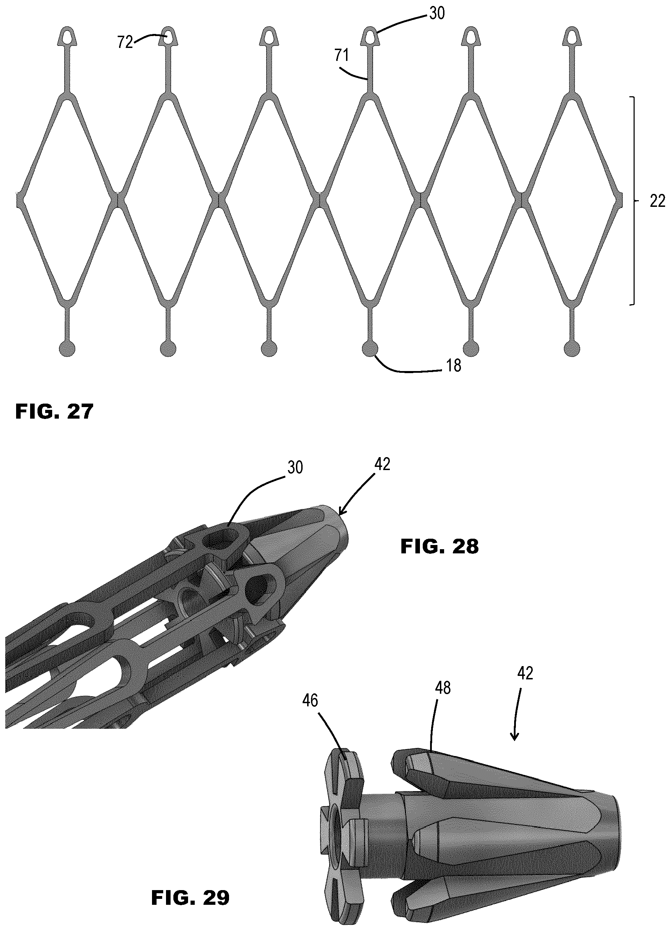

Figs. 27-28 show an embodiment using attachment elements 30 having a mushroom-head shape (also referred to as a helmet-shape). The shaping may provide a more atraumatic profile of the attachment elements, and/or offer a larger contact area between the attachment elements 30 and the stent holder 42. Such shape and position of the attachment elements at the very end of extensions or stalks 71 facilitates the radial compression by the retrieval feature 70 allowing funnel-like shaping of the outflow end of the prosthesis providing easy engagement of the retrieval catheter during the prosthesis retrieval. The stent holder 42 may comprise a segmented front wall 46 and a rear wall 48 defining a form fit accommodation for the attachment elements 20, to prevent relative axial movement in both directions. The rear wall 48 also has a ramp profile tapering away from the segmented front wall 46. The ramp profile may reduce risk of the stent holder 42 catching undesirably on anatomical tissue and/or an introducer sheath when the delivery catheter is subsequently withdrawn.

Figs. 30-32, 73 and 80 show embodiments using attachment elements 30 having a diamond shape. Such a shaping may be even more atraumatic than a mushroom-head shape. The shaping may also reduce any risk of the attachment elements snagging a retrieval filament (described hereinafter). The segmented front wall 46 and rear wall 48 of the stent holder are shaped to define a form fit around the attachment elements in a similar manner to Figs. 28 and 29. In this example, both the segmented front wall 46 and the rear wall 48 of the stent holder 42 have ramp profiles, thereby reducing the risk of the stent-holder catching undesirably on anatomical tissue in use, and also providing a self-ramping or guiding effect with respect to other components which may slide against or in contact with the stent-holder.

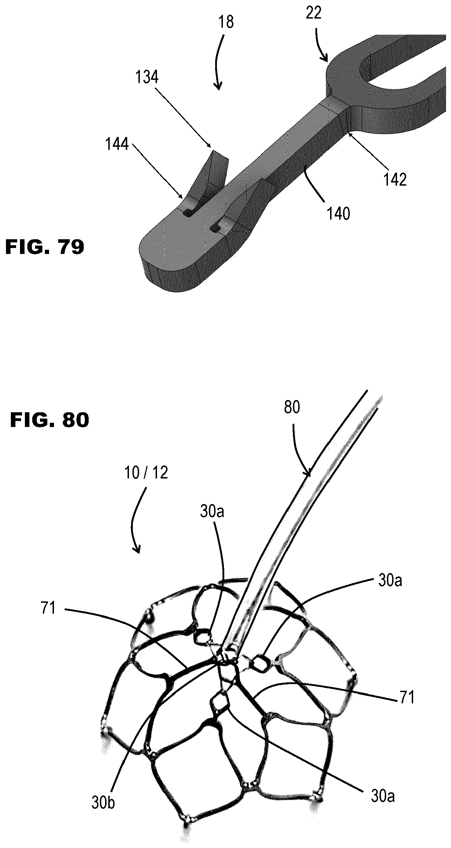

In the example shown in Figs 73 and 80, the attachment elements 30 are disposed in at least: a first sub-set 30a of at least one attachment element, but here three elements; and a second sub-set 30b of at least one attachment element, but here three elements. The first and second sub-sets 30a and 30b are disposed at respective different first and second positions along an axis of the stent, at least in the collapsed condition of the stent (and optionally also in the expanded condition). The first sub-set of attachment elements 30a may be disposed adjacent to the trunk portion 22 of the stent. The second sub-set of attachment elements 30b may be carried on struts or stalks 71 to be axially offset further away from the trunk portion 22.

The first and second sub-sets 30a and 30b of attachment elements may be generally adjacent to, but axially offset from, one another in the axial direction. Referring to Fig. 80, offset attachment elements and/or attachment features may facilitate collapsing the stent to a smaller diameter, than were all of the attachment elements and/or attachment features to be arranged at the same axial position. Offsetting can avoid the attachment elements from interfering with one another when the stent is converted to or held in its collapsed condition. Looking from a different perspective, it can also enable a larger number and/or larger individual size of attachment elements and/or attachment features to be used without compromising the diameter of the stent when in its collapsed condition. If desired, the first and second sub-sets may have a shape or configuration that interfit with respect to one another, and /or nestle together, when in the collapsed condition. The disposition of attachment elements may alternate in the circumferential direction between the first and second sub-sets 30a and 30b, such that each attachment element of the first sub-set 30a is disposed circumferentially between two attachment elements of the second sub-set 30b. More than two sub-sets 30a and 30b could be used if desired. Although this mixed arrangement has been illustrated with the specific example of diamond-shaped attachment elements, the disposition of attachment elements 30 in at least first and second sub-sets may be used with any form of attachment element including, for example, the other forms of attachment elements described herein.

In all of the above embodiments and whether or not shown explicitly, the attachment elements 30 (if provided) may optionally be canted inwardly, to reduce the risk of the attachment elements 30 damaging surrounding anatomical tissue, and to facilitate engagement of the attachment elements with a stent holder during delivery. The inwardly canted shape may also facilitate retrieval and collapsing to the collapsed condition by presenting an initial taper to facilitate sliding a collapsing sheath over the prosthesis starting at the attachment elements. Optional inward canting may also be advantageous for use with a retrieval element, facilitating hooking of the prosthesis as described later.

Various possibilities exist for the design of the valve component 14. It may be of a mono-leaflet type, or a multi-leaflet type (for example, bi-leaflet or tri-leaflet). The valve component 14 may be made of a single piece of material, e.g. folded into a suitable tubular form, or from separate leaflets assembled together.

The valve component 14 may be constructed of suitable flexible biocompatible material, for example, biological tissue (for example, pericardial tissue such as porcine pericardial tissue or bovine pericardial tissue), and/or synthetic material in web form (for example, woven web or non-woven web) or film form, or a combination of both. Example synthetic materials include PET and polyurethane.

Figs. 4 and 7 illustrate a bi-leaflet valve component 14 comprising two saddle-shaped pockets of material attached (e.g. sutured) to each other at opposite ends of a mutual diameter, leaving a central portion unattached and mobile to form a pressure-responsive unidirectional valve. The pockets are orientated within the stent 12 with their closed ends towards the prosthesis inflow end, and their open ends towards the prosthesis outflow end. When blood pressure is higher at the inflow end, blood pushes the mobile portions of the pockets apart, opening the central portion to permit forward blood flow. When blood pressure is higher at the outflow end, blood fills the pockets, pressing the mobile portions together to close the central portion and block reverse blood flow.

Figs. 5 and 6 illustrate a windsock-style valve component 14 having its wide mouth orientated towards the inflow end of the prosthesis, and its narrow aperture towards the outflow end. The windsock functions in a similar manner to the bi-leaflet valve described above. Forward flow into the wide end of the windsock forces the flexible sides apart to open the valve to the forward flow. Reverse flow from the outlet end collapses the flexible sides of the windsock, closing the central aperture, and blocking the reverse flow.

Figs. 8-9 and 11-12 illustrate a valve component 14 comprising leaflets 24 attached directly to struts of the stent 12, e.g. by suturing along their peripheries by sutures 25. The term valve component 14 refers to the leaflets 24 collectively whether or not the leaflets 24 are attached directly to one another. The valve component of Figs. 8-9 comprises three leaflets 24, and the valve component of Figs. 11-12 comprises two leaflets 24. The leaflets present slack portions that behave as a pressure-responsive unidirectional valve. When the blood pressure at the inlet is higher, the leaflets are forced apart to open the central region to forward blood flow. When the blood pressure at the outlet is higher, the leaflets collapse against each to block reverse through the valve.

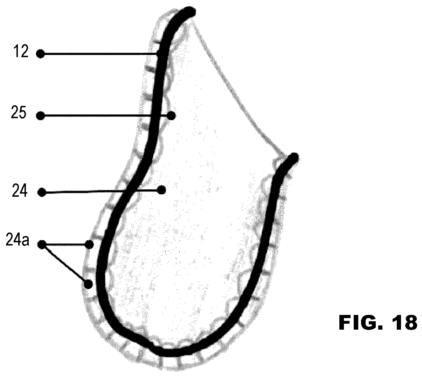

Referring to Fig. 18, in some examples, the leaflets 24 may be larger than the stent, such that a peripheral portion 24a of each leaflet 24 extends radially beyond the exterior surface of the stent 12. The peripheral portion 24a can provide a seal for improving sealing against the surrounding anatomical tissue. The peripheral portion 24a may optionally be divided into segments that are flexible independently of one another, to enhance the conformability of the seal to the surrounding anatomy.

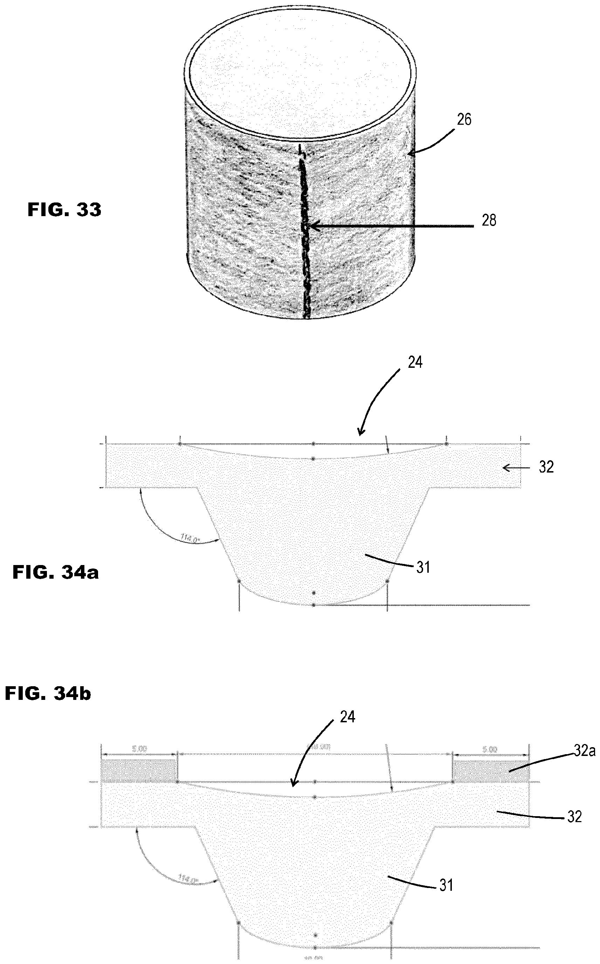

Referring to Figs. 33-44, in an alternative form, the valve component 14 comprises leaflets 24 mounted to a tubular conduit 26. The conduit 26 may be made of the same material as the leaflets 24, or of different material. The conduit 26 may serve to prevent blood leakage between the leaflets 24 and the stent 12, and/or to separate the leaflets 24 from the stent 12, and/or to distribute the support for the leaflets 24 with respect to the stent structure, to reduce risk of undesirable force concentrations on the leaflets 24.

Figs.33-44 illustrate example constructions of the valve component 14 with conduit 26. Referring to Fig. 33, the conduit 26 may be formed initially from a sheet of material rolled into tubular form, and attached (e.g. sutured) along an axial seam 28.

Referring to Figs. 34a and 34b, the leaflets 24 are cut individually from sheet material. Each leaflet comprises a belly portion 31 with a cusp edge, an opposite coaptation edge, and two commissural side tabs 32. Two different options for the tabs are illustrated: in Fig. 34a, the tabs 32 are generally co-extensive with the top coaptation edge of the leaflet; in Fig. 34b, the tabs 32 include additional material 32a extending above the top coaptation edge and used for additional reinforcement, as described below.

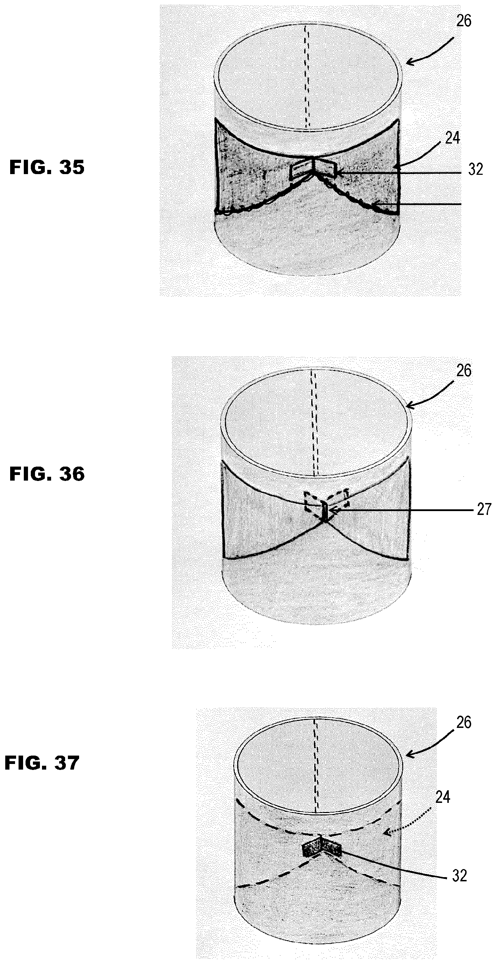

Referring to Fig. 35, the cut leaflets 24 are assembled to the conduit 26 in everted (or inside-out) form, that is to say that the leaflets 24 are attached (e.g. sutured) along their cusp edges to the exterior of the conduit 26, leaving the tabs 32 unattached.

Referring to Fig. 36, a slit 27 is made in the conduit at the level of the tabs 32, allowing the tabs 32 to be pushed through the slit to project into the interior of the conduit 26.

Referring to Fig. 37, the conduit 26 is folded to de-evert the assembly, transferring the leaflets 24 into the interior of the conduit 26, and the tabs 32 now projecting outwardly to the exterior.

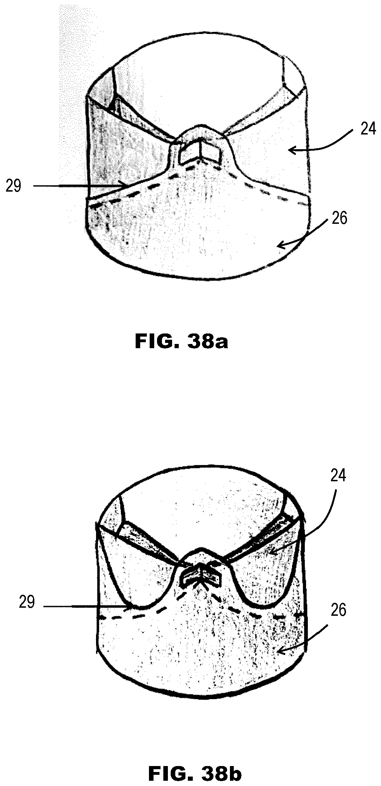

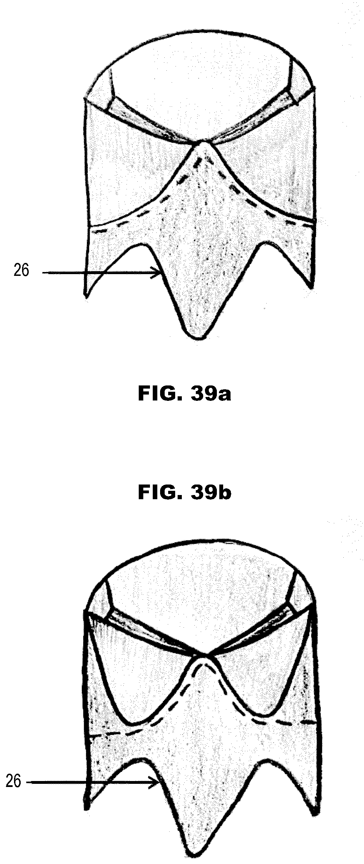

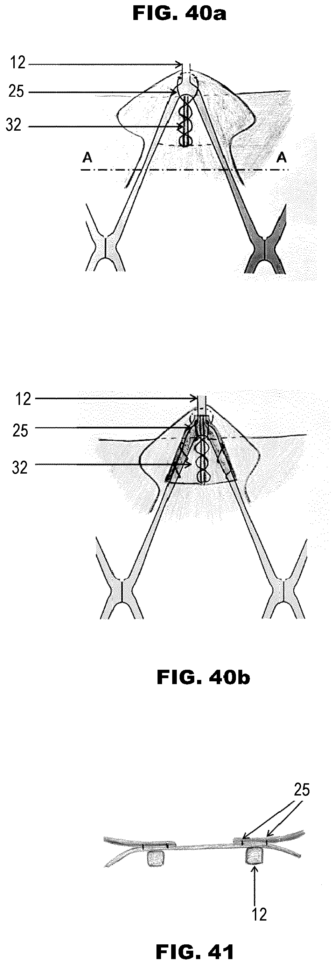

Figs. 38 and 39 illustrate optional trimming stages for the conduit 26, to reduce the bulk of the conduit material, and/or permit collapsing to a smaller size, and/or to reduce risk of the conduit 26 blocking tributary vessels of the pulmonary veins. Figs. 38a and 38b illustrate two options for trimming the upper (outflow) edge 29 of the conduit 26. In the option of Fig. 38a, the conduit 26 is trimmed to match the general profile of the leaflets and commissures, defining three commissure peaks separated by three scallops following the leaflet cusp edge. In the option of Fig. 38b, the conduit 26 is trimmed to define six commissure peaks, ie. the three commissure peaks of Fig. 38a plus an additional three intermediate peaks. These six peaks match the upper six apexes of the stent 12. Although not shown in Figs. 38a-b in detail, the commissure peaks are trimmed to include excess flap-like material (or ears) around the commissures (visible in Figs. 40a and 40b).

Figs. 39a and 39b depict optional trimming of the inflow edge of the conduit 26, to define six extensions matching the lower six apexes of the stent 12. Fig. 39a depicts the lower trimming of the conduit of Fig. 38a, and Fig. 39b depicts the same lower trimming of the conduit of Fig. 38b.

After trimming (if used), the valve component 14 may be assembled to the stent 12. Referring to Figs. 40a and 40b, the valve component 14 may be attached at its commissures by means of a suture 25 passing through the tabs 32 and wrapped around the corresponding apex of the stent 12. The suture 25 directly supports the commissures by means of the tabs 32. The strut of the attachment element provides additional fixation to ensure that the commissure attachment cannot slip from the stent apex. Fig. 40a depicts the attachment when the leaflet geometry of Fig. 34a is used, and Fig. 34b depicts the attachment for the leaflet geometry of Fig. 34b. For the latter, the additional material 32a of the tab 32 can be folded over the struts at the apex to provide additional reinforcement of the commissure attachment.

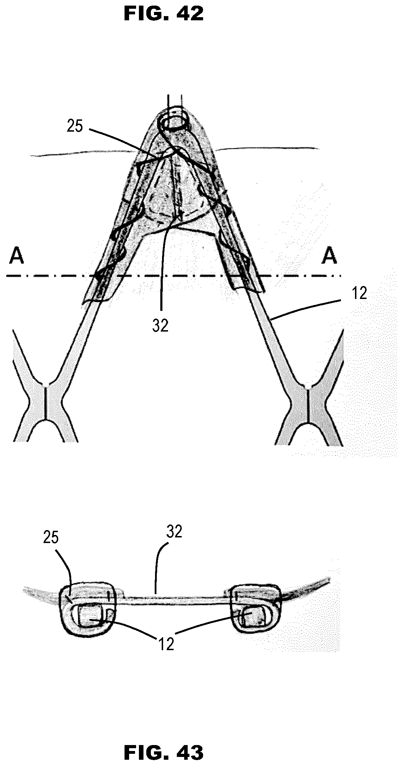

Referring to Fig. 42, the flap-like excess material of the conduit 26 at the commissures can be folded over the struts in the region of the apex, and sutured, to reinforce and protect the attachment of the tabs 32.

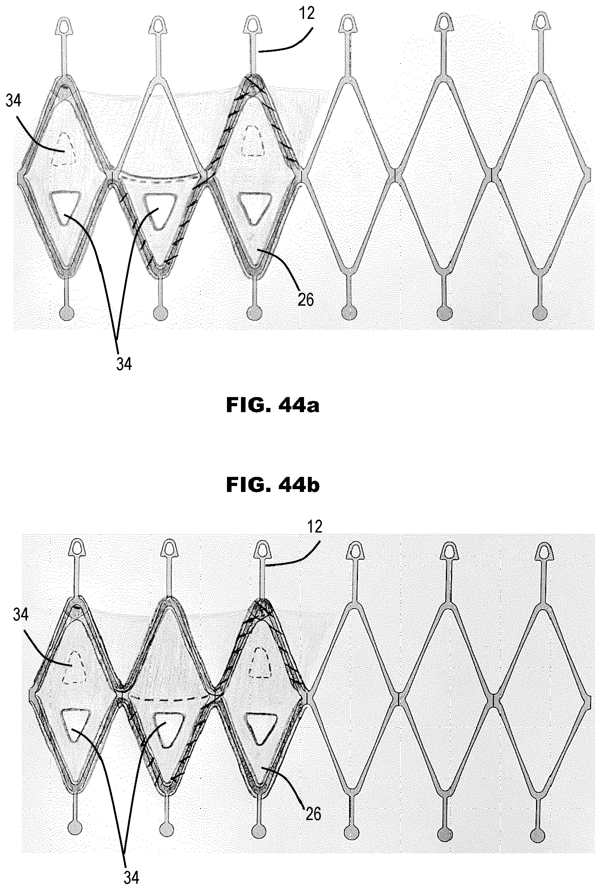

Figs. 44a and 44b illustrate additional attachment of the valve component 14 via its conduit 26 to secure the valve component 14 to the stent 12. The conduit 26 is sutured along the struts defining the diamond-shaped cells, where the conduit is co-extensive with the struts. Fig. 44a depicts the attachment for the conduit trimmed according to Fig. 39a, with six lower (inflow) extensions matching the six lower apexes of the stent cells, and the three upper (outflow) peaks matching every second upper apex of the stent cells. Fig. 44b depicts the corresponding attachment for the conduit trimmed according to Fig. 39b, with six inflow extensions and six outflow peaks.

Optionally, one or more apertures 34 may be formed (e.g. cut) in the conduit 26 where the conduit defines a panel spanning or partly spanning a cell of the stent 12. The apertures 34 may reduce risk of the conduit 26 blocking tributary vessels of the pulmonary veins, when implanted.

In all of the different examples of valve component 14, additional wash-through apertures 56 (Figs. 8 and 9) may be provided in regions of the leaflets 24 or other parts of the valve component that are positioned away (or furthest away) from direct blood flow. Wash-through apertures 56 may permit limited flow of blood to bypass the leaflets, and wash-out these regions that might otherwise not be subjected to flow. It is believed that wash-through apertures 56 may be effective in avoiding thrombosis formation in such areas. As mentioned earlier, thrombosis formation is a problem that prosthetic mitral valves have experienced in some cases. Although wash-through apertures 56 are only illustrated for the example in Figs 8 and 9, it is explicitly envisaged that such apertures may be used in corresponding positions (e.g. near the base of the leaflet cusp) of all of the valve components 14 described herein to benefit from the same advantages.

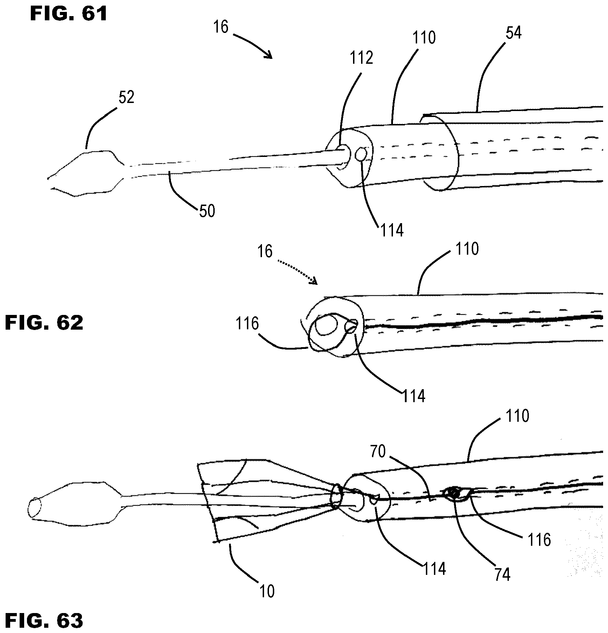

A further feature of the prosthetic valves 10 in some examples disclosed herein, is the provision on the valve 10 of a retrieval feature 70 configured to facilitate retrieval of an implanted valve 10 after it has been deployed (and optionally separated) from a delivery catheter. For example, the valve 10 may be retrieved at some later time for removal or replacement. As explained later with respect to Figs. 61-70, the retrieval feature can also be used to facilitate the valve placement into delivery system and attachment to delivery catheter as well as the valve release from delivery catheter during implantation. Using the flexible retrieval element 70 during implantation can provide the slow release of the outflow part of the stent-valve and prevent jumping of the latter and consequent mal-positioning. In addition, in case of suboptimal position of the valve in the vein (or in another valvular position, for example, aortic, mitral) before the complete disconnection and release, the retrieval feature can allow easy recapturing of the valve into delivery system and repositioning. Therefore the retrieval feature can allow for easier release, recapturing and possibility to reposition of the stent-valve during the implantation procedure as well as providing a possibility to retrieve the implanted valve after (e.g. up to several weeks after) the implantation. These functions of the retrieval feature make its use very beneficial for pulmonary venous, aortic (TAVI) and mitral (TMVR) applications.

Optionally, the retrieval feature 70 may be made of or comprise radiopaque material to facilitate identification using medical imaging (e.g. X-ray imaging), and to facilitate guiding a retrieval catheter into engagement with the retrieval feature to grasp the valve.

Also optionally, the retrieval feature 70 is provided on a portion of the prosthesis that, in use, projects from the ostium into the left atrium (Figs. 2 and 3). Such a configuration can facilitate coupling the retrieval catheter to each valve 10 in turn by manipulating the distal end of the retrieval catheter in the atrium.

As already described, the examples shown in Figs. 4 and 5 include a hub 13 that can act as a retrieval feature. Using a retrieval catheter (not shown) with a suitable snare or hub connector, the retrieval catheter may engage the hub 13 and apply tension while a collapsing sheath is advanced over the valve 10 to draw the valve back to a collapsed configuration.

Figs. 15 and 16 illustrate an embodiment using deployable retrieval features 70 integrated with the attachment elements 30. The retrieval features 70 comprise elements (e.g. tabs or fingers), optionally cantilever mounted at one end, that can fold flat with and/or into the attachment elements when the stent 12 is compressed by a compressing force to its collapsed configuration. This gives the stent, in particular the attachment element, a smooth profile when compressed (Fig. 17) without interfering with the function of the attachment element 30 to engage the stent holder 42, nor occupying excessive space. Upon expansion of the stent, the element 70 self-expands to its deployed state projecting outwardly from the surface of the attachment element 30. The free end of the element 70 may point generally towards the inflow end of the stent and/or away from the attachment element 30. This provides a hookable feature that can be engaged by a snare or a stent-catcher of a retrieval catheter, allowing tension to be applied to the stent from the outflow end while a collapsing sheath is slid over the body of the stent to collapse the stent to its collapsed configuration.

As shown in Fig. 14, the attachment elements 30 may be mounted on, or take the form of stalks 71, for example, springy stalks that extend from apexes of the stent trunk portion and converge to a small diameter "∅D" at the outflow extremity of the stent 12, thereby presenting the elements 70 in close proximity to each other to facilitate retrieval.

Referring to Figs. 6, 8-13, 19-32 and 45-47, the retrieval feature 70 may be provided in the form of at least one flexible filament. The flexible filament may pass through one or more dedicated apertures 72 in the stent 12. In some of the drawings, the filament 70 is not shown explicitly, but its presence is implicit by virtue of dedicated apertures 72 for the filament 70. The flexible filament may present one or more exposed elongate sections, for example, suspended between the attachment elements 30 or other struts, suitable for being hooked for retrieval. The flexible retrieval element can also be lassoed to its free end left floating in the aperture of the outflow part of the stent-valve. In some embodiments the free end can be advantageously equipped with a small bead facilitating the lassoing.

Different configurations of flexible filament are envisaged for different valve designs. The flexible filament may be configured such that applying tension to the filament, for example in an axial direction, tends to contract at least a portion of the stent 12 adjacent to the filament, to facilitate drawing the prosthesis into a retrieval catheter. Referring to Figs. 10 and 13, for example, the filament may be suspended at plural positions around the circumference of the prosthesis. Pulling the filament in an axial direction (optionally while countering axial force on the prosthesis) tensions the filament, and tends to draw the plural positions circumferentially and/or radially closer to one another. Multiple filaments 70 may also be provided, for example, at different axial positions along the prosthesis. As illustrated below with reference to Figs. 61-70, a specially designed delivery catheter can allow pulling multiple filaments separately whereby allowing independent actuation of distal, middle and proximal sections of the stent-valve. Such possibility could provide important advantages for pulmonary veins, TAVI and TMVR applications.

The filament may be positioned at or towards an outflow end of the prosthesis 10. In the case of a pulmonary vein prosthesis, the outflow end corresponds to the end that is closest to and/or may extend partly within, the ostium and/or the left atrium.

The flexibility of the filament 70 may avoid the filament 70 from interfering with the collapsibility of the prosthesis 10.

Depending on the configuration of the filament 70, the filament 70 may have or present a generally closed-loop or endless shape, which may be achieved by knotting together or adjacent each other the ends of a length of filament. Alternatively, the filament may have or present distinct ends which are spaced apart from one another, and held captive with respect to the stent by means of knots.

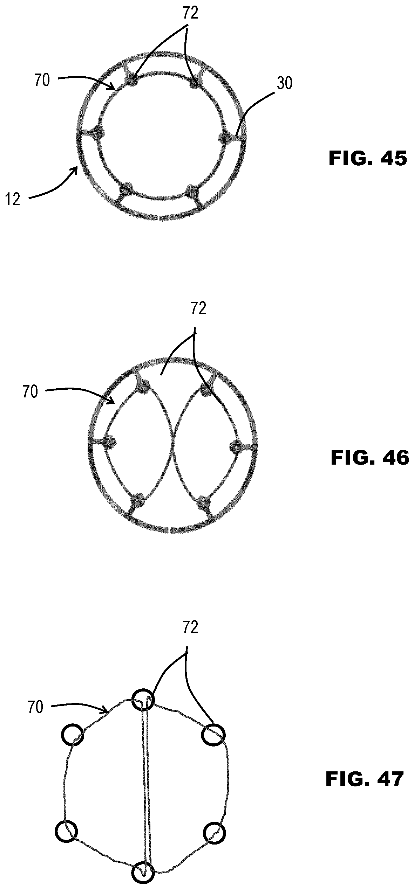

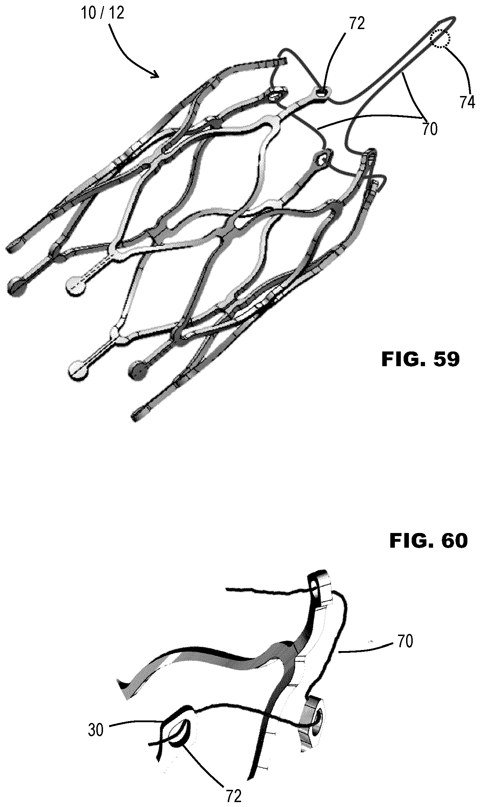

Figs. 6, 45 and 59 illustrate the filament 70 extending entirely circumferentially with respect to the stent. The filament 70 may have any desired length depending on the desired characteristics. For example, in Fig. 45, the filament 70 has a length generally corresponding to the (e.g.) stent circumference in the expanded condition. In Fig. 6, the filament 70 has some slack. In Fig. 59, the filament 70 has significant slack. The two parts of the filament forming the slack could be braided in a tress or knotted to facilitate capturing by the lasso of the delivery catheter. In Figs. 45 and 59, the attachment elements 30 are optionally canted inwardly with respect to the trunk portion 22, thereby providing space radially inwardly and outwardly of the filament 70 to permit access by a retrieval catheter. The inwardly canted shape may also facilitate retrieval and collapsing to the collapsed condition by presenting an initial taper to facilitate sliding a collapsing sheath over the prosthesis starting at the attachment elements.

Figs. 10 and 13 illustrate the filament 70 extending radially with respect to the stent. In Fig. 13, the filament extends diametrically between diametrically opposed apexes of the stent 12. The filament 70 has knotted ends 73 that cannot pass through the apertures 72, thereby maintaining the filament captive. Optionally, a radiopaque bead 74 may be carried by the filament 70 to facilitate hooking or lassoing by a retrieval catheter. Fig. 10 illustrates a similar filament presenting a tristar shape suspended between three apexes. A bead 74 (e.g. radiopaque) may also optionally be provided in other embodiments (e.g. Fig. 59) if desired to provide an element on the filament 70 that can be caught or gripped by a retrieval catheter and/or by a manipulation element of a delivery catheter. The bead 74 may also be placed at a distal end of the slack of the filament 70 (Fig. 59) to facilitate its lassoing by the delivery and/or retrieval catheters.

Figs. 46 and 47 illustrate the filament 70 extending partly circumferentially and partly radially, to provide multiple different sections extending in different directions available for hooking by a retrieval catheter. The filament 70 may have a generally figure-of-8 configuration with respect to the apertures 72, optionally presenting a crossover at the centre (Fig. 46).

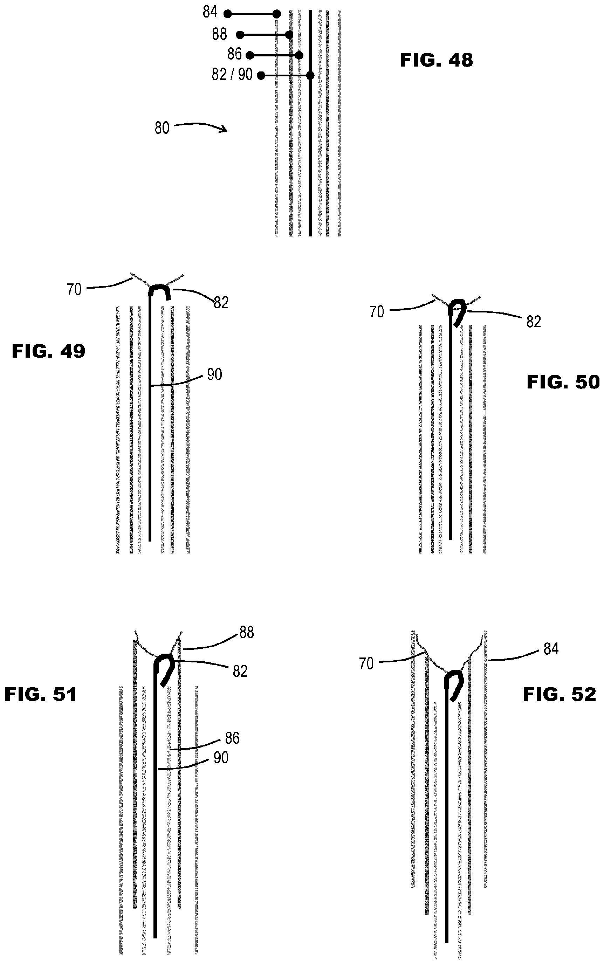

Figs. 48 to 58 illustrate examples of retrieval catheter 80 for retrieving cardiovascular implants, especially but not limited to cardiovascular valves. The retrieval catheters 80 are especially suitable for retrieving any of the valves described herein.

The retrieval catheter may comprise a catcher 82 for catching on a retrieval feature (70) of the implant, and at least one sheath 84. The sheath 84 and the catcher 82 are displaceable axially with respect to one another between a first condition (e.g. Figs. 49) in which the catcher 82 extends axially beyond a mouth of the sheath 84 for catching on a prosthetic valve, and a second condition (e.g. Fig. 52) in which the catcher 82 resides within the sheath 84 for retrieving the prosthetic valve 10 with respect to the sheath.

In the examples, the catcher 82 can change shape between a stowed configuration (Fig. 48) in which the catcher 82 is initially set for introduction in the catheter, an open configuration (Fig. 49), and a closed configuration (Figs. 50). The closed configuration may be the same as the stowed configuration, or it may be different (as Figs. 48 and 50).

In the examples, plural sheaths are provided for controlling the catcher 82.

For example, in Fig. 48, the retrieval catheter 80 further comprises a control sheath 86 and a locking sheath 88. The control sheath 86 is a narrow sheath surrounding an elongate shaft 90 forming part of, or carrying, the catcher 82. The catcher 82 comprises or is made from shape-memory wire formed into a curled shape. The configuration of the wire can be controlled by the degree to which shaft 90 is advanced with respect to the control sheath 86, and how much of the wire is unconstrained by the control sheath 86. When the shaft 90 is withdrawn (Fig. 48), the wire is constrained to a relatively straight shape (stowed configuration). By advancing the shaft 90 with respect to the control sheath 86, the exposed portion of the wire begins to curl to define an open hook (open configuration, Fig. 49). In this configuration, the catcher 82 can be hooked onto the retrieval feature 70 of an implant. By further advancing the shaft 90, the wire completes its curl to an almost closed shape (closed configuration, Fig. 50), capturing the retrieval feature 70.

The locking sheath 88 may then be advanced (Fig. 51) with respect to the shaft 90 and the control sheath 86 to surround the catcher 82, and prevent the catcher 82 from uncurling when tension is applied subsequently.

Thereafter, while retaining the shaft 90 the control sheath 86 and the locking sheath 88 relatively stationary (optionally under tension), the sheath 84 may be advanced (Fig. 52) to slide over the implant and progressively collapse the implant into the sheath 84. Applying tension to the catcher 82 may draw the end of the implant (e.g. stent 12) into a narrower diameter, thereby facilitating sliding the sheath 84 over the implant. Once the sheath 84 has been slid over the implant, the retrieval catheter may be withdrawn from the body containing the collapsed implant.

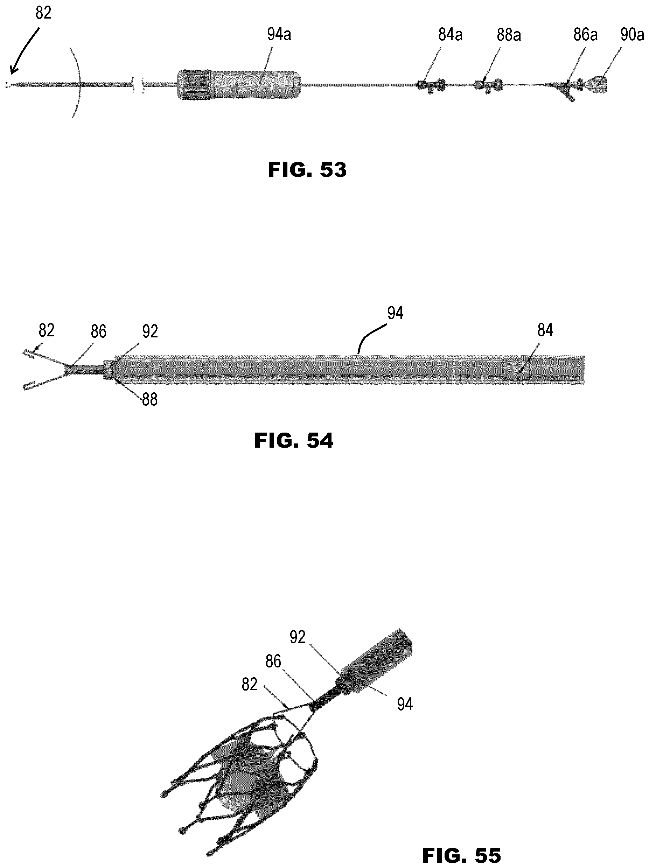

Figs. 53 to 58 show modifications of the retrieval catheter of Figs. 48 to 52. The same reference numerals are used to denote equivalent features. The suffix "a" is added to represent a handle portion coupled to the respective sheath and/or shaft, for manipulating the respective member. Any combination of one or more of the modifications may be implemented, as desired.

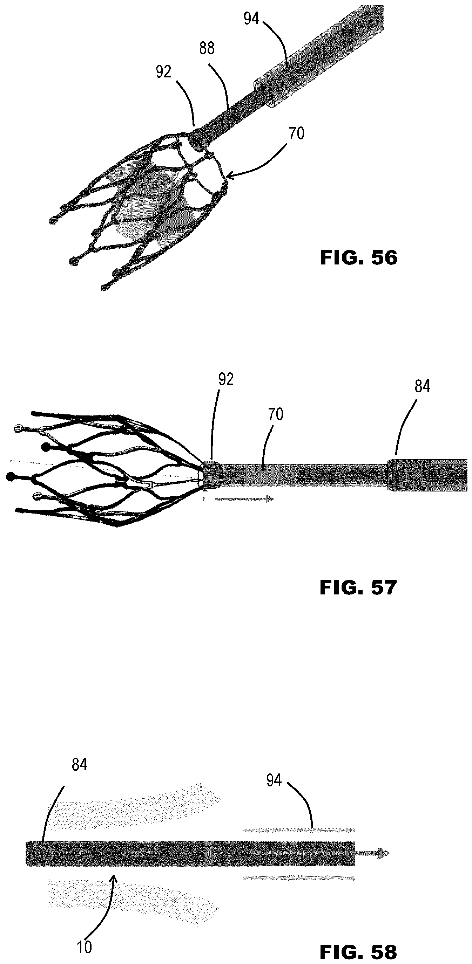

One of the modifications is that instead of the catcher comprising a single element, the catcher 82 comprises plural elements, in this example, two elements. The two elements form a pair of jaws configured to (i) open (e.g. diverge) when the catcher 82 is extended with respect to the control sheath 86 and/or locking sheath 88, and (ii) to close (e.g. narrow) when the catcher 82 is withdrawn relative to the control sheath 86 and/or locking sheath 88 (see Fig. 57). The use of plural jaws may make it easier to catch on to a prosthetic valve than using a single catcher element.

A second modification is that the locking sheath 88 has an enlarged mouth 92. The mouth may, for example, be funnel shaped, or step-shaped, or flared. The enlarged mouth 92 may act as a guide to facilitate drawing the end of the implant (e.g. stent 12) into a narrow diameter shape, as best seen in Fig. 57.

A further modification used in this example is the provision of a guide sheath 94 through which the other tubes and elements 82-92 are fed. The guide sheath 94 may be steerable via a handle 94a to enable the retrieval catheter to be guided to a site of an implant to be retrieved.

In addition to, or alternatively to, the filament 70 serving as a retrieval feature of the prosthesis, in some embodiments the filament 70 may serve to control expansion and/or collapsing of the prosthesis 10 with respect to the delivery catheter 16, for example, for one or more of: controlled collapsing of the prosthesis on to the catheter (e.g. loading) for introduction into the body; controlled expansion of the prosthesis for deployment; controlled collapsing of the prosthesis during deployment to permit repositioning and/or removal. This is illustrated in the following examples.

Referring to Figs. 61-63, the delivery catheter 16 may comprise (e.g. similarly to the embodiment of Fig. 22), at least at a prosthesis accommodation region at a distal end, an inner shaft or tube 50, an atraumatic tip 52 carried by the inner shaft 50, and a sheath 54 displaceable axially with respect to the inner shaft 50 for selectively covering or uncovering the prosthesis accommodation region. The delivery catheter 16 may further comprise an intermediate tube 110 nested within the sheath 54, and having a first channel (e.g. lumen) 112 accommodating the inner shaft 50, and a second channel (e.g. lumen) 114 accommodating a manipulation device 116 for manipulating the flexible filament 70 of the prosthesis 10. If the first lumen 112 is offset to one side of a central axis of the delivery catheter 16, the atraumatic tip 52 mounted on the inner tube 50 may optionally similarly be offset on the inner tube 50 such that the atraumatic tip 52 may remain generally coaxial with the sheath 54.

In the illustrated example (Fig. 62), the manipulation device 116 comprises a snare, a hook or lasso. The manipulation device 116 is axially movable with respect to the second lumen 114. Referring to Fig. 62, by advancing the manipulation device 116 to project beyond the second lumen 114, the manipulation device 116 can enlarge to receive, or release, the filament 70 of the prosthesis. Referring to Fig. 63, by retracting the manipulation device 116 into the second lumen 114, the snare of the manipulation device 116 may be tightened to grip the filament 70, optionally snaring the bead 74 (if provided). Further retraction of the manipulation device 116 may apply tension to the filament 70 thereby to collapse the prosthesis 10 from one end, towards a funnel shape. Thereafter, the prosthesis 10 can be collapsed entirely, by sliding the sheath 54 distally over the partially collapsed prosthesis 10. Optionally, a loading funnel (not shown) may be fitted temporarily to ease collapsing of the prosthesis during the loading operation.

Once the catheter has been introduced to the implantation site, deployment of the prosthesis 10 may be similar to be above, in reverse order. The sheath 54 may be slid axially proximally, allowing the prosthesis 10 to expand progressively from the distal end (with respect to the catheter), to the partially-expanded funnel shape constrained at its opposite end. In this partially-expanded state, the function and/or positioning of the prosthesis 10 may optionally be assessed by the practitioner, using medical imaging as described above. If the practitioner is ready to continue deployment, the manipulation device 116 may be advanced distally in the second lumen 114, to relax the tension in the filament 70, and allow the prosthesis 10 to deploy fully towards its expanded configuration. Once the snare of the manipulation device 116 projects from the mouth of the second lumen 114, the snare may release the grip on the filament 70, thereby disengaging the prosthesis 10. If at any time during this process the practitioner desires to re-collapse the prosthesis 10, this may be achieved easily and reliably by retracting the manipulation device 116, and advancing the sheath 54 distally over the prosthesis.

Although not shown explicitly in the drawings, a stent holder 42 (e.g. similar to any of the examples described above) may optionally be provided on the delivery catheter 16, to positively retain attachment elements 30 of the prosthesis in fixed axial position with respect to the delivery catheter 16, while at least the end of the prosthesis 10 with the attachment elements 30 is constrained in its collapsed shape (e.g. by the sheath 54 and/or by tension from the manipulation device 116).

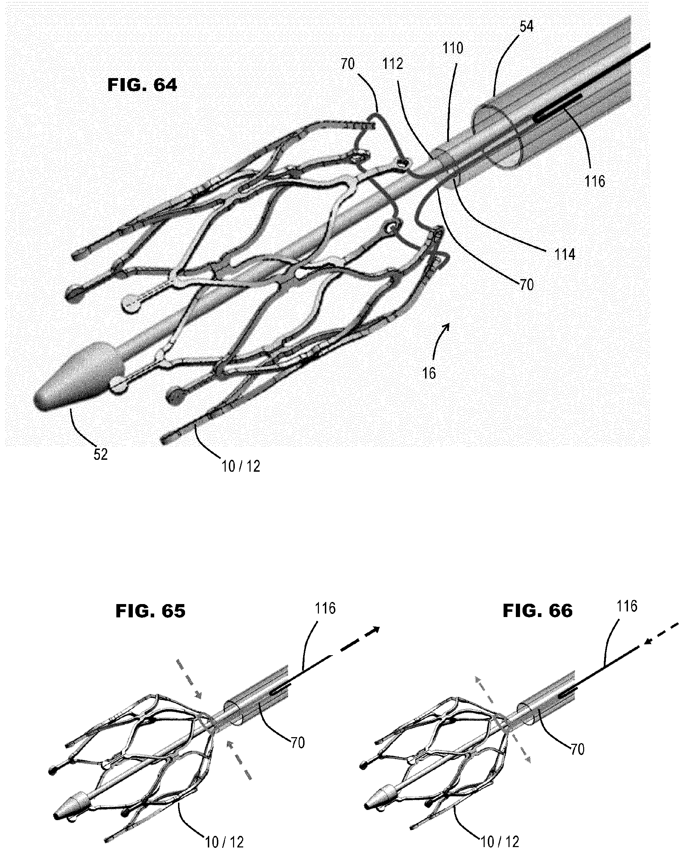

Figs. 64-66 illustrate a further example of delivery catheter, similar to Figs. 61-63 except that the manipulation device 116 comprises a hook that engages the filament 70 by hooking in the axial direction. The functionality is very similar to the preceding embodiment. Axial retraction of the manipulation device 116 (Fig. 65) applies tension to the filament, drawing the adjacent end of the prosthesis radially inwardly towards a partially collapsed configuration. Axial advancement of the manipulation device 116 (Fig. 66) relaxes tension in the filament 70, allowing the end of the prosthesis to expand, and allowing the hook to release from the filament 70 to disengage the prosthesis. Also, although not shown explicitly in Figs. 64-66, a stent holder 42 (e.g. similar to any of the examples described above) may optionally be provided on the delivery catheter 16, to positively retain attachment elements 30 of the prosthesis in fixed axial position with respect to the delivery catheter 16, while at least the end of the prosthesis 10 with the attachment elements 30 is constrained in its collapsed shape (e.g. by the sheath 54 and/or by tension from the manipulation device 116).

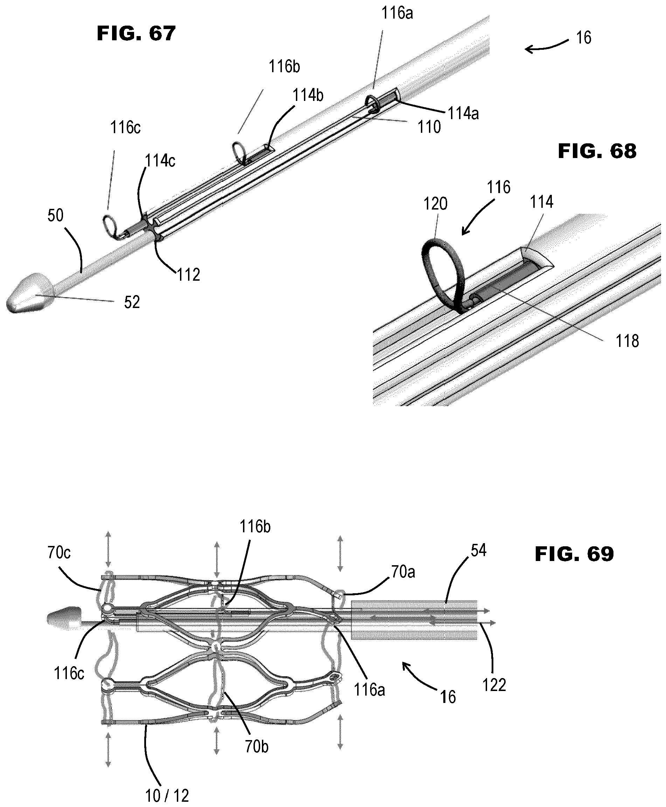

Although the preceding embodiments illustrate a prosthesis with a single flexible filament 70, two, three or more filaments 70 may be provided as desired, optionally at different positions along the axial length of the prosthesis 10 and/or stent 12. For example, Figs. 69, 70 and 72 illustrate a prosthesis 10 (the valve component is omitted to avoid obscuring other details) having three filaments 70a, 70b and 70c. The first filament 70a is positioned towards one end (outlet end), in a similar manner to the filament 70 described in earlier examples. The second filament 70b is provided towards the centre, and the third filament 70c is provided towards an opposite end (inlet end) to the first filament 70a. The plural filaments enable control over expansion/collapsing of the prosthesis over its length, and not merely at one end.

Figs. 67-71 illustrate a delivery catheter 16 for controlling expansion/contraction of the prosthesis 10 by means of the plural filaments 70a-c. The delivery catheter 16 comprises a respective manipulation device 116a-c for each filament 70a-c, three manipulation devices 116a-c in the present example.

Referring to Fig. 67, the delivery catheter 16 comprises an intermediate tube 110 having multiple (e.g. four) channels, for example, in the form of lumen 112 and 114a-c. A first lumen 112 receives an inner tube 50 carrying atraumatic tip 52. Providing the inner tube 50 as a separate item from the intermediate tube 110 allows the length of the stent accommodation region to be adjusted or configured to suit different lengths of prostheses, and/or to retract the atraumatic tip 52, for example, after implantation. However, in some embodiments (not shown), the inner tube 50 may alternatively be integral with the intermediate tube 110, rather than being a separate item received within a dedicated lumen 112. Alternatively, the intermediate tube 110 itself may extend to the atraumatic tip 52 without any small diameter inner tube segment. In such cases the length of the segment housing the prosthesis may be specific to the prosthesis diameter/length, rather than being configurable by use of a separate inner tube 50.

Three additional lumens 114a-c receive the three manipulation devices 116a-c. Each lumen 114a-c has a window opening at a different axial position in the prosthesis accommodation region, to provide the respective manipulation devices 116a-c at an appropriate axial position for the respective filament 70a-c of the prosthesis. Optionally, the window may have the form of the lumen opening on one side to form an open channel, or open slot, extending axially.

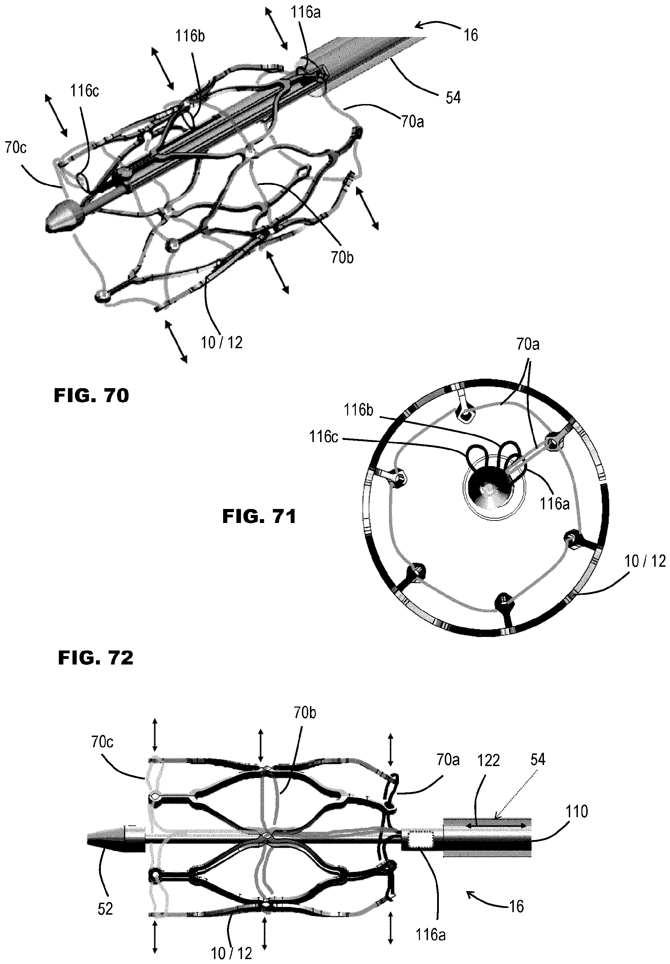

The lumen 112 and 114a-c may be angularly distributed around a central axis of the intermediate tube 110. The atraumatic tip 52 may be offset radially on the inner tube 50 such that the atraumatic tip 52 is generally concentric with the intermediate tube 110 and/or with an optional sheath 54 (Figs. 69 and 70). In the embodiments of Figs. 67 to 72 the sheath 54 may be optional because the valve may effectively be compressed over its entire length by the flexible filaments 70a-70c. The openings for the lumen 114 for the manipulation devices 116 may be angularly offset or distributed with respect to one another (e.g. in addition to being axially offset or distributed with respect to one another), such that the manipulation devices 116 are angularly distributed (e.g. in addition to being axially distributed).

Referring to Fig. 68, each manipulation device 116 comprises a snare or lasso or hook 120 coupled to a hypotube 118. To facilitate capturing/lassoing of the flexible elements 70a-70c the distal end of the manipulation device could be mildly bent outwardly (not shown). Referring to Figs. 67-71, the manipulation devices 116a-c are controlled by axial movement of the snare 120 and/or hypotube within the intermediate tube 110, as represented by the arrows 122. Retracting the snare 120 and/or hypotube 118 tightens the snare 120 to capture the filament 70, and apply tension to the filament 70 to collapse the portion of the prosthesis corresponding to the filament 70. Advancing the snare 120 and/or hypotube 118 relaxes tension applied to the filament 70 allowing the portion of the prosthesis 10 corresponding to the filament 70 to self-expand. Continued advancement of the snare 120 and/or hypotube ultimately allows the snare 120 to open to release the filament 70 from the snare 120. The manner in which the manipulation device 116a interacts with the filament 70a is illustrated in Fig. 71, the other filaments 70b and 70c being omitted in this figure to avoid obscuring the detail of the filament 70a.

In some embodiments, the manipulation devices 116a-c may be operable independently, for example, by respective actuators (not shown) at a handle of the delivery catheter 16. This may permit manual control over the order in which the different portions of the prosthesis are expanded. Alternatively, in some embodiments, the manipulation devices 116a-c may be operable collectively, for example, by a collective actuator (not shown) at a handle of the delivery catheter 16. The collective actuator could be configured to operate the manipulation devices 116 simultaneously, or in a predetermined overlapping and/or non-overlapping operating sequence.

In a similar manner to that described previously, the manipulation devices 116a-c may be operated to perform any one or more of: initial collapsing or loading of the prosthesis on to the delivery catheter prior to introduction into the body; partial expansion of the prosthesis at an implantation site; complete or near complete expansion of the prosthesis at an implantation site; recollapsing of the prosthesis from the partially expanded or expanded state, if desired for repositioning or withdrawal; optional reexpansion of a recollapsed prosthesis; release of an expanded prosthesis from the delivery catheter.

The delivery catheter 16 may optionally comprise a sheath 54 slidable over the prosthesis accommodation region. The sheath 54 may act as a constraining sheath to supplement the manipulation devices 116 and the filaments 70, and/or as a protection sheath to protect the prosthesis once loaded at the prosthesis accommodation region.

Fig. 72 shows use of a further example of delivery catheter 16 for a prosthesis 10 having three filaments 70a-c distributed along the length of the prosthesis, as described above. The delivery catheter 16 is very similar to that described in the preceding embodiment, except that the filaments 70 couple releasably to a single manipulation device (shown schematically at 116) coupled to the intermediate tube 110. Axial movement of the intermediate tube 110, as represented by the arrows 122, moves the manipulation device 116, which correspondingly applies tension to, or relaxes tension in, the filaments 70. The filaments 70 are manipulated simultaneously with each other. Optionally, by controlling the length of each filament 70a-c, the point at which tension is applied/released can be set relative to the other filaments to provide a sequential effect.

In the above embodiments, the filament(s) 70 and/or 70a-c remain an integral part of the prosthesis 10, even after implantation. Therefore, it is not necessary to pull on the filaments 70 to remove them after deployment, nor is it necessary to provide removal channels in the prosthesis for allowing the sutures to be pulled out. This can provide a more stable implantation procedure than were the filaments intended to be withdrawn by being pulled on after deployment, and it can also simplify the design of the prosthesis. Moreover, the presence of at least one filament permanently on the prosthesis can act as a retrieval feature facilitating later retrieval of the prosthesis after implantation.

Figs. 73 to 79 illustrate embodiments including modified configurations of contact projection 18, in the form of an antimigration element 18 having a shape (i) to resist migration (at least in one axial direction) of the stent 12 after implantation, and (ii) configured to as not to protrude radially outwardly compared to an outer surface of the trunk portion 22 adjacent the antimigration element 18. In Fig. 74, broken line 132 represents a level of the outer surface of the stent adjacent to the antimigration member 18. Optionally, a radially outermost antimigration feature, e.g. a tip of, e.g. a bard or prong 134, of the antimigration element 18 lies radially inwardly, by at least a certain distance, of the stent outer surface 132. The radial distance may, for example, be at least or greater than a radial thickness of the struts. Additionally, or alternatively, the radial distance may be at least 0.1 mm, optionally at least 0.2mm, optionally at least 0.3mm, optionally at least 0.4mm, optionally at least 0.5mm, optionally at least 0.6mm, optionally at least 0.7mm, optionally at least 0.8mm, optionally at least 0.9mm, optionally at least 1mm.

In some embodiments, plural antimigration elements 18 are positioned in an annular pattern at the extremity of the trunk portion 22, e.g. at an extremity of the stent or the prosthesis. Each antimigration element 18 is individually coupled (e.g. integrally) to the adjacent stent structure. The antimigration element 18 may project axially from the stent structure that supports it. With such an arrangement, the antimigration element 18 maintains a fixed relation with the adjacent stent structure. When the stent structure is compressed to its collapsed condition, the antimigration elements also compress with the adjacent stent structure. The antimigration elements 18 may have the radially non-protruding configuration in both the compressed condition (Fig. 75) and the expanded condition (Fig. 73) of the stent.

Referring to Fig. 75, configuring the antimigration element 18 such that it does not protrude radially outwardly compared to the outer surface of the trunk portion 22 adjacent the antimigration element (and optionally is spaced radially inwardly by at least a certain distance), avoids the antimigration element 18 interfering with operation of a delivery catheter 16 for introducing the prosthesis to an implantation site, and/or a retrieval catheter 80 for retrieving and/or re-sheathing the prosthesis after implantation. As described above, such a catheter 16 or 80 may comprise at least one sheath 54 that slides directly over and in contact with the prosthesis outer surface in its collapsed condition. Problem-free operation of the catheter 16 or 80 may rely on the sheath being able to slide, without too much friction between the outer surface of the stent 12 and an inner surface 54a of the sheath 54. Despite the antimigration element 18 having a shape intended to resist relative movement, the antimigration element 18 is maintained clear of the inner surface 54a of the sheath 54.

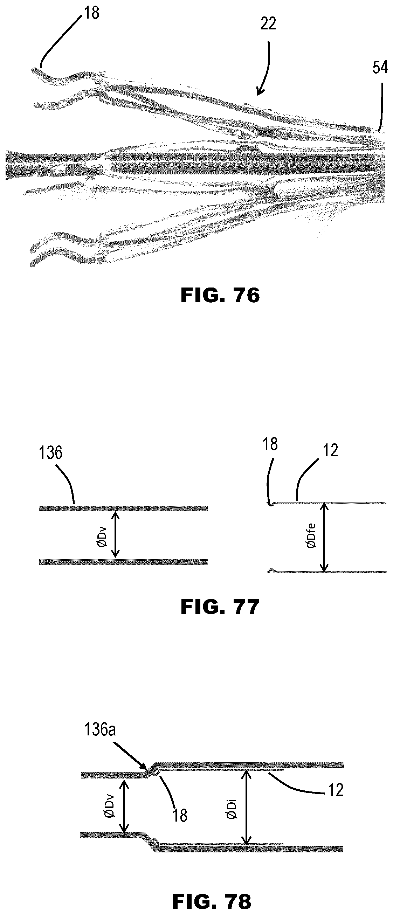

When the sheath 54 is retracted (to the right in Figs. 75 and 76) to allow the stent to expand, the antimigration elements 18 do not displace radially outwardly until the sheath has begun to clear the adjacent stent structure (e.g. the trunk portion), allowing the stent to expand. Similarly, when the sheath 54 is advanced over the stent (to the left in Figs. 75 and 76) for collapsing or re-collapsing the stent, the antimigration elements 18 shift radially inwardly ahead of the sheath, as the sheath compresses the stent structure that supports the antimigration elements 18. The result is that the sheath 54 generally does not need to make substantial contact with the antimigration elements 18, and the antimigration elements 18 do not interfere with smooth sliding movement of the sheath 54, during sheathing, unsheathing or re-sheathing of the stent for deployment and/or recapture and/or retrieval after implantation.

Fig. 76 depicts the stent 12 in a partially deployed state, in which the sheath 54 has been retracted sufficiently to allow the stent 12 to expand from one end at which the antimigration elements 18 are carried, while the other end of the stent remains constrained by the sheath, and attached to the catheter by the attachment elements 30. During implantation, the antimigration elements 18 may be the first portions of the stent 12 that contact surrounding anatomical tissue, to stabilize the stent during implantation, and to engage the tissue in a manner that assists anchoring.

Although the antimigration elements 18 do not protrude radially proud of the outer surface of the trunk portion 22, the antimigration elements are effective to assist anchoring by resisting migration of the stent in at least one axial direction. Various effects are possible, as follows, independently or two or more in combination.

For example, referring to Fig. 76, for implantation in a blood vessel having a generally elastic tubular wall 136 (e.g. a pulmonary vein) with an inner diameter ∅Dv, a self-expanding stent 12 would be used having a slightly larger diameter ∅Dfe (for example, oversize by between about 1 and 10mm). Referring to Fig. 77, upon implantation, the stent 12 expands to an intermediate diameter ∅Di, when the outward force from the stent 12 is in equilibrium with the elastic return force of the vessel wall. The vessel wall becomes "tented". In other words, the vessel wall adopts a slightly stretched shape defined by the stent, like material of a tent stretched over a tent-frame. The antimigration elements 18 engage the vessel wall 136 at an extremity 136a of the tented portion where the vessel wall transitions from its normal diameter ∅Dv to its tented diameter defined by the stent.

Additionally or alternatively, when a prosthesis is implanted into an elastic blood vessel, the struts of the stent can embed somewhat into the surface of the vessel wall, thereby bringing the antimigration elements 18 into engagement with the tissue of the vessel wall.

Additionally or alternatively, an ostium of a pulmonary vein flares slightly at the junction with the atrium, and narrows away from the atrium. This provides a natural anatomical situation in which antimigration elements 18 at the end of the prosthesis engaging the narrowing tissue will also naturally engage the with the anatomical tissue to assist anchoring.

Various shapes of antimigration element 18 are envisaged. The antimigration element 18 may, for example, comprise an antimigration feature, for example, a barb and/or prong 134 (Figs. 74 and 79), for penetrating at least partly into anatomical tissue in contact with the antimigration member when the prosthesis is implanted. In the illustrated examples, the antimigration element 18 may comprise a cantilever support 140 having a first coupled end 142, coupled to (e.g. extending from) the trunk portion 22 of the stent 12. The cantilever support 140 may be canted, or otherwise project, at least partly radially inwardly away from the first coupled end 142. The feature 134 may be carried at a second free end 144 of the cantilever support and be bent, curved or angled radially outwardly compared to the cantilever support 140. The inward canting of the cantilever support 140 can provide sufficient radial space to accommodate the radial height of the barb or prong 134, without the barb or prong 134 projecting radially proud of the outer surface of the trunk portion 22.

In the examples of Figs. 73-78, the bard or prong 134 points at least partly in a axial direction away from the trunk portion 22 of the stent 12. In the alternative example of Fig. 79, the barb or prong 134 points at least partly in an axial direction towards the trunk portion 22 of the stent. Fig. 79 also illustrates plural barbs or prongs 134 within the same antimigration feature. In each case, the barb or prong 134 is configured to resist migration at least in the direction in which the barb or prong 134 points. The barb or prong 134 resists dragging by tending to embed deeper into the anatomical tissue in contact. The antimigration element 18 can therefore be configured to provide migration resistance in one or both directions, independently of the respective end of the stent at which the antimigration element 18 is provided.

It will be appreciated that the forgoing description is merely illustrative and does not limit the scope of protection. Many modifications and improvements may be made within the scope and/or principles of the invention.

## Claims

1. A pulmonary vein prosthesis, comprising:
a stent component, and
a valve component;
the stent component and the valve component being configurable in a collapsed configuration for delivery by catheter to a pulmonary vein implantation site, and an expanded configuration for implantation in a pulmonary vein.

2. The prosthesis of claim 1, wherein the stent component is of self-expanding material, the prosthesis further comprising a retrieval feature of the prosthesis for facilitating recollapsing and retrieval of the prosthesis from the implantation site, by means of a retrieval catheter, after implantation.

3. The prosthesis of claim 1 or 2, wherein the stent component is of self-expanding material, the prosthesis further comprising a flexible filament carried by the stent component, the flexible filament being permanently captive to the stent component, and optionally wherein:
(i) the flexible filament optionally configured such that application of external tension to the filament tends to draw a portion of the prosthesis radially inwardly, to facilitate recollapsing of the prosthesis;
and/or
(ii) the flexible filament optionally extends through apertures of the stent component.

4. The prosthesis of claim 3, wherein the stent component comprises a plurality of attachment elements for removable engagement with a stent holder, the attachment elements carrying the flexible filament.

5. The prosthesis of claim 3 or 4, wherein the stent component further comprises a plurality of individual contact projections extending from a trunk portion of the stent component towards an inlet end of the stent component.

6. The prosthesis of any preceding claim, wherein the valve component comprises a plurality of coapting leaflets, each leaflet comprising a mobile coaptation region and a cusp region opposite to the coaptation region, wherein at least a portion of the cusp region extends beyond the stent component to provide at least one seal extension, optionally at least two seal extensions, for sealing against a vessel wall.

7. A transcatheter cardiovascular stent-valve prosthesis, optionally according to any preceding claim, comprising:
a stent component made of self-expanding material, and
a valve component;
the stent component and the valve component being configurable in a collapsed configuration for delivery by catheter to a cardiovascular implantation site, and an expanded configuration for implantation in a cardiovascular system;
the prosthesis further comprising at least one flexible filament carried by the stent component and being permanently captive to the stent component, the flexible filament including at least one portion exposed for engagement by a manipulating device for applying tension to the flexible filament, the flexible filament being engaged with the stent component such that a configuration of at least a portion of the stent component is controllable in response to the applied tension,
optionally wherein there are at least two flexible filaments arranged at respective different positions along the length of the stent component, optionally wherein there are at least three flexible filaments arranged at respective different positions along the length of the stent component.

8. The prosthesis of claim 3 or any claim dependent thereon, or claim 7, wherein:
(i) the flexible filament has a shape, in at least one configuration of the stent component, selected as one or more of: at least partly circumferential with respect to a portion of the stent component; at least partly radial with respect to a portion of the stent component; a figure-of-eight shape with respect to the stent component;
and/or
(ii) the flexible filament has a closed loop shape.

9. The prosthesis of claim 3 or any claim dependent thereon, or claim 7 or 8, wherein the flexible filament is disposed towards an end of the stent component, optionally towards an outflow end with respect to the valve component.

10. The prosthesis of claim 3 or any claim dependent thereon, or any of claims 7 to 9, wherein the flexible filament comprises or carries a feature that can be gripped or caught, optionally a bead, to facilitate engagement of the filament by a manipulating device.

11. A retrieval catheter for retrieving a deployed cardiovascular prosthesis, optionally a prosthesis according to any preceding claim, the retrieval catheter comprising:
a catcher for catching on a retrieval feature of the prosthesis, and
at least one sheath;
the catcher and the sheath being displaceable axially with respect to one another between a fist condition in which the catcher extends axially beyond a mouth of the sheath for catching on a prosthetic valve, and a second condition in which the catcher resides within the sheath for retrieving the prosthetic valve with respect to the sheath;
optionally wherein said at least one sheath includes first and second sheaths disposed one within another and axially slidable with respect to each other and with respect to the catcher.

12. The catheter of claim 11, wherein:
(i) the catcher is configured to change configuration between at least two of:
a stowed condition for introduction;
an open condition for catching on to the retrieval feature of the prosthetic valve;
a closed condition for capturing the retrieval feature with respect to the catcher
and/or
(ii) the catcher comprises at least one selected from: a hook; a barb; a snare; a lasso; a pair of jaws.

13. A delivery catheter for implanting a cardiovascular prosthesis of a type comprising at least one flexible filament that is part of the prosthesis for controlling expansion and/or collapsing of at least a portion of the prosthesis, the prosthesis optionally of a type according to any of claims 1 to 10, the delivery catheter comprising:
at least one shaft having a prosthesis accommodation region at or towards a distal end, and
a manipulation device extending from the proximal end to the distal end of the shaft, the manipulation device comprising an engagement element at the stent accommodation region for releasably engaging the flexible filament of the prosthesis, and an actuator for applying or relaxing tension in the flexible filament, thereby to control expansion and/or collapsing of at least a portion of the prosthesis.

14. The delivery catheter of claim 13, comprising a plurality of engagement elements for engaging a respective plurality of filaments of the prosthesis, and optionally wherein the delivery catheter further comprises a plurality of actuators for actuating the engagement elements individually.

15. A combination of:
at least one prosthesis according to any of claims 1 to 10 for implantation in at least one respective pulmonary vein; and
a space-occupying member for implanting into a left atrium for reducing the reservoir volume of the left atrium between the pulmonary veins and a native mitral valve.
